# EUROPEAN PATENT SPECIFICATION

(11) **EP 3 028 047 B1**
(45) Date of publication and mention of the grant of the patent: **03.06.2020**
(21) Application number: 14832653.1
(22) Date of filing: 31.07.2014
(51) Int. Cl.: G01N 33/574, G01N 33/68

(54) **COMPOSITIONS AND METHODS FOR IDENTIFYING A RISK OF CANCER IN A SUBJECT**
ZUSAMMENSETZUNGEN UND VERFAHREN ZUR IDENTIFIZIERUNG DES RISIKOS VON KREBS BEI EINEM MENSCHEN
COMPOSITIONS ET PROCÉDÉS D'IDENTIFICATION D'UN RISQUE DE DÉVELOPPEMENT D'UN CANCER CHEZ UN PATIENT

(30) Priority: 31.07.2013 US 201361860669 P; 07.06.2014 US 201462009175 P; 08.07.2014 US 201462021998 P
(43) Date of publication of application: 08.06.2016
(73) Proprietor: University of Miami, Miami, Florida 33136 (US)
(72) Inventor: FRANZMANN, Elizabeth, Miami, Florida 33136 (US)
(74) Representative: Dehns
(86) International application number: PCT/US2014/049223
(87) International publication number: WO 2015/017692

(56) References cited:
- WO-A1-2010/060103
- WO-A1-2013/074793
- WO-A1-2013/074793
- US-A1- 2007 190 529
- US-A1- 2012 115 165
- US-A1- 2012 115 165
- Lola Duffort: "University of Miami scientists are developing a 'rinse and spit' test for oral cancers", Miami Herald, 23 May 2014 (2014-05-23), XP055359578, Retrieved from the Internet: URL:http://www.miamiherald.com/living/arti cle1964660.html [retrieved on 2017-03-28]
- ELIZABETH J. FRANZMANN ET AL: "Salivary protein and solCD44 levels as a potential screening tool for early detection of head and neck squamous cell carcinoma", HEAD & NECK, vol. 34, no. 5, 11 May 2012 (2012-05-11), pages 687-695, XP055069517, ISSN: 1043-3074, DOI: 10.1002/hed.21810
- SEBASTIAN MAYER ET AL: "Increased soluble CD44 concentrations are associated with larger tumor size and lymph node metastasis in breast cancer patients", JOURNAL OF CANCER RESEARCH AND CLINICAL ONCOLOGY, SPRINGER, BERLIN, DE, vol. 134, no. 11, 26 April 2008 (2008-04-26), pages 1229-1235, XP019658254, ISSN: 1432-1335, DOI: 10.1007/S00432-008-0397-Z

## Description

### ACKNOWLEDGEMENT OF GOVERNMENT SUPPORT

This invention was made with government support under Grant Nos. R01CA118584 and RO3 CA107828 awarded by the National Institutes of Health. This invention was also made with support under Bankhead-Coley Grant No. 10BG02 from the Florida Department of Health. The government has certain rights in the invention.

### BACKGROUND

Head and neck squamous cell carcinoma (HNSCC) is a debilitating and deadly disease marked by disparities, with a mortality rate in blacks twice that of whites. It is more common in men than women (Cancer Facts and Figures-2013. Atlanta: American Cancer Society, 2013). HNSCC accounts for almost 90% of cancers involving the upper aerodigestive tract (UADT) (Muir et al. Upper aerodigestive tract cancers. Cancer Suppl (1995) 75:147-53). Each year the disease affects 50,000 people in the United States and 600,000 people world-wide. Survival rates are poor because most patients present in late stage when cure rates are as low as 30% (Vokes, et al. Head and neck cancer. N Engl J Med (1993) 328:184-94). The disease can be cured 80-90% of the time if detected early (Markopoulos, et al. Salivary Markers for Oral Cancer Detection. Open Dent J. (2010) 4:172-178).

Many of the current HNSCC biomarker studies use "omics" approaches to identify a few candidate markers from a large pool of potential markers using case-control study design (Shankar, et al. Trends in salivary diagnostics - a 5-year review of oral oncology 2007-2011. Oral Oncol (2012) 48:e22-3; Li, et al. Salivary transcriptome diagnostics for oral cancer detection. Clin Cancer Res (2004) 10:8442-8450; Park, et al. Salivary microRNA: discovery, characterization, and clinical utility for oral cancer detection, Clin Cancer Res (2009) 15:5473-5477; Hu, et al. Clin Cancer Res (2008) 14:6246-6252; Carvalho, et al. Evaluation of promoter hypermethylation detection in body fluids as a screening/diagnosis tool for head and neck squamous cell carcinoma. Clin Cancer Res (2008) 14:97-107; Elashoff, et al. Prevalidation of salivary biomarkers for oral cancer detection. Cancer Epidemiol Biomarkers Prev (2012) 21(4):664-72). On further testing, the markers usually fail to perform as well. For example, panels of methylation markers yielded sensitivity of 35-85% and specificity of 30-90% (Carvalho, et al. Evaluation of promoter hypermethylation detection in body fluids as a screening/diagnosis tool for head and neck squamous cell carcinoma. Clin Cancer Res (2008) 14:97-107) while a panel of mRNA markers yielded sensitivity of 45-79% and specificity of 72-77% (Elashoff D, et al. Prevalidation of salivary biomarkers for oral cancer detection. Cancer Epidemiol Biomarkers Prev (2012) 21(4):664-72).

The most important predisposing factors for the development of HNSCC are tobacco, alcohol and human papillomavirus (HPV) infection, usually with HPV type 16 (Muscat, et al. Tobacco, alcohol, asbestos, and occupational risk factors for laryngeal cancer. Cancer (1992) 69:2244-51; Blot, et al. Smoking and drinking in relation to oral and pharyngeal cancer. Cancer Res (1988) 48:3282-7; Burch, et al. Tobacco, alcohol, asbestos, and nickel in the etiology of cancer of the larynx: a case-control study. J Natl Cancer Inst (1981) 67:1219-24; Johnson N. Tobacco use and oral cancer: a global perspect. J Dental Edu (2001) 65:328-339; Balaram P, et al. Oral cancer in southern India: the influence of smoking, drinking, paan-chewing and oral hygiene. Int J Cancer (2002) 98:440-45; Lewin F, et al. Smoking tobacco, oral snuff and alcohol in the etiology of squamous cell carcinoma of the head and neck. Cancer (1998) 82:1367-75; Mashberg A, et al. Tobacco smoking, alcohol drinking, and cancer of the oral cavity and oropharynx among U.S. veterans. Cancer (1993) 72:1369-75; Talamini R, et al. Cancer of the oral cavity and pharynx in nonsmokers who drink alcohol and in nondrinkers who smoke tobacco. J Natl Cancer Inst (1998) 90:1901-3; Lin BM, et al. Long-term prognosis and risk factors among patients with HPV-associated oropharyngeal squamous cell carcinoma. Cancer. (2013); D'Souza G, et al. Case-control study of human papillomavirus and oropharyngeal cancer. N Engl J Med (2007) 356:1944-56; Gao J, et al. Basic consideration of research strategies for head and neck cancer. Front Med 2012;6:339-53). Tobacco is a strong risk factor for head and neck cancer and acts synergistically with alcohol use to increase risk (Muscat, et al. Tobacco, alcohol, asbestos, and occupational risk factors for laryngeal cancer. Cancer (1992); 69:2244-51; Blot, et al. Smoking and drinking in relation to oral and pharyngeal cancer. Cancer Res (1988) 48:3282-7; Burch, et al. Tobacco, alcohol, asbestos, and nickel in the etiology of cancer of the larynx: a case-control study. J Natl Cancer Inst (1981) 67:1219-24; Johnson. Tobacco use and oral cancer: a global perspect. J Dental Edu (2001) 65:328-339; Balaram, et al. Oral cancer in southern India: the influence of smoking, drinking, paan-chewing and oral hygiene. Int J Cancer (2002) 98:440-45; Lewin, et al. Smoking tobacco, oral snuff and alcohol in the etiology of squamous cell carcinoma of the head and neck. Cancer (1998) 82:1367-75; Mashberg, et al. Tobacco smoking, alcohol drinking, and cancer of the oral cavity and oropharynx among U.S. veterans. Cancer (1993) 72:1369-75; Talamini, et al. Cancer of the oral cavity and pharynx in nonsmokers who drink alcohol and in nondrinkers who smoke tobacco. J Natl Cancer Inst (1998) 90:1901-3; Lin, et al. Long-term prognosis and risk factors among patients with HPV-associated oropharyngeal squamous cell carcinoma. Cancer. 2013; Anantharaman, et al. Population attributable risk of tobacco and alcohol for upper aerodigestive tract cancer. Oral Oncology (2011) 47:725-31). HPV-associated HNSCC is one of the few cancers that is increasing in incidence in the United States. While tumors that are driven primarily by HPV have an excellent prognosis, the majority of HPV tumors occur in individuals with a history of smoking (Lin, et al. Long-term prognosis and risk factors among patients with HPV-associated oropharyngeal squamous cell carcinoma. Cancer. 2013; Ang, et al. Human papillomavirus and survival of patients with oropharyngeal cancer. N Engl J Med (2010) 363(1):24-35). HPV positive tumors in smokers have a worse prognosis (Lin, et al. Long-term prognosis and risk factors among patients with HPV-associated oropharyngeal squamous cell carcinoma. Cancer. 2013; Kumar, et al. EGFR, p16, HPV Titer, Bcl-xL and p53, sex, and smoking as indicators of response to therapy and survival in oropharyngeal cancer. J Clin Oncol (2008) 26:3128-37).

The current "gold standard" for screening is physical examination followed by biopsy, but sensitivity is only 64% and specificity is 74% (Brocklehurst, et al. Screening programmes for the early detection and prevention of oral cancer. Cochrane Database Syst Rev (2010) 11 :CD004150). A variety of technologies exist to aid in the detection of oral cancers, but there is no evidence that they are any better than the naked eye for screening. The development of a "rinse and spit" test for oral cancers is described in Duffort, 2014 (University of Miami scientists are developing a "rinse and spit" test for oral cancers. Miami Herald, 23 May 2014, www.miamiherald.com/living/article1964660.html). What is needed in the art is a method of identifying a risk of cancer, detecting cancer, providing a prognosis of cancer, or monitoring cancer progress during treatment. The subject matter disclosed herein addresses these and other needs.

### SUMMARY

The invention provides a method of determining a risk of head and neck squamous cell cancer in a subject using a kit comprising: saline solution; a cup for receiving an oral saline rinse; at least one antibody that specifically binds CD44; a reagent for determining total protein concentration; and reference levels for soluble CD44 (solCD44) and total protein, the method comprising:
a) measuring a test amount of solCD44 in a sample of an oral saline rinse obtained from the subject in the cup, using the at least one antibody that specifically binds CD44;
b) measuring a test amount of total protein in the sample using the reagent for determining total protein concentration; and
c) determining the risk of head and neck squamous cell cancer in the subject by determining whether the test amount of solCD44 and the test amount of total protein are above or below the reference levels of solCD44 and total protein, wherein the reference levels of solCD44 are about 2.2 ng/mL and about 5.3 ng/mL and the reference levels of total protein are about 1.2 mg/mL and about 0.6 mg/mL, wherein the reference levels of solCD44 and total protein delimit different, statistically significant risks for the cancer and wherein the term about means within 5% of the specified value.

Additional advantages will be set forth in part in the description that follows and the Figures, and in part will be obvious from the description, or may be learned by practice of the aspects described below. The advantages described below will be realized and attained by means of the elements and combinations particularly pointed out in the appended claims. It is to be understood that both the foregoing general description and the following detailed description are exemplary and explanatory only and are not restrictive.

### BRIEF DESCRIPTION OF THE FIGURES

The accompanying Figures, which are incorporated in and constitute a part of this specification, illustrate several aspects of the invention and together with the description serve to explain the principles of the invention.
Figure 1 shows targeting CD44 by siRNA inhibits tumor growth, EGFR expression and phosphorylation in CAL 27. Figure 1A shows a tumor growth curve of CAL 27 and its CD44-siRNA stable transfectant (3C3). Figure 1B shows immunostaining of tumor sections (20X).
Figures 2A and 2B show progression-free survival (PFS) in 137 clinic-based case cohort is lower in patients with high solCD44 levels (>10 ng/mL) (Figure 2A) and protein levels (>1 ng/mL) (Figure 2B). The negative effect of high solCD44 levels is seen in each racial ethnic group studied, blacks, white Hispanics (WH), and white non-Hispanics (WNH) (Figure 2C).
Figure 3 shows the distribution of differences in CD44 measurements taken one year apart.
Figures 4A and 4B show the cervical PAP smear results in controls when measuring solCD44 (Figure 4A) and protein levels (Figure 4B).
Figures 5A-F shows Kaplan-Meier Curves demonstrating significant differences in PFS (Figure 5A) and OS (Figure 5B) based on CD44 and protein level cutpoints. The differences in CD44 (Figure 5C) and protein (Figure 5E) levels over 1 year follow a normal distribution. Linear regression analysis shows that the trend towards decreasing levels over one year is significant for both CD44 (Figure 5D) and protein (Figure 5F).

### DETAILED DESCRIPTION

The compositions and methods described herein may be understood more readily by reference to the following detailed description of specific aspects of the disclosed subject matter and the Examples and Figures included therein.

Before the present compositions and methods are disclosed and described, it is to be understood that the aspects described below are not limited to specific synthetic methods or specific reagents, as such may, of course, vary. It is also to be understood that the terminology used herein is for the purpose of describing particular aspects only and is not intended to be limiting.

### General Definitions

In this specification and in the claims that follow, reference will be made to a number of terms, which shall be defined to have the following meanings:
Throughout the description and claims of this specification the word "comprise" and other forms of the word, such as "comprising" and "comprises," means including but not limited to, and is not intended to exclude, for example, other additives, components, integers, or steps.

As used in the description and the appended claims, the singular forms "a," "an," and "the" include plural referents unless the context clearly dictates otherwise. Thus, for example, reference to "a composition" includes mixtures of two or more such compositions, reference to "an agent" includes mixtures of two or more such agents, reference to "the component" includes mixtures of two or more such components, and the like.

"Optional" or "optionally" means that the subsequently described event or circumstance can or cannot occur, and that the description includes instances where the event or circumstance occurs and instances where it does not.

Ranges can be expressed herein as from "about" one particular value, and/or to "about" another particular value. By "about" is meant within 5% of the value, e.g., within 4, 3, 2, or 1% of the value. When such a range is expressed, another example includes from the one particular value and/or to the other particular value. Similarly, when values are expressed as approximations, by use of the antecedent "about," it will be understood that the particular value forms another example. It will be further understood that the endpoints of each of the ranges are significant both in relation to the other endpoint, and independently of the other endpoint.

As used herein, by a "subject" is meant an individual. Thus, the "subject" can include domesticated animals (*e.g.,* cats, dogs, etc.), livestock (*e.g.,* cattle, horses, pigs, sheep, goats, etc.), laboratory animals (*e.g.*, mouse, rabbit, rat, guinea pig, etc.), and birds. "Subject" can also include a mammal, such as a primate or a human. The terms "subject" and "patient" are used interchangeably throughout the application.

"Marker" or "biomarker" are used interchangeably herein and refer to a polypeptide (of a particular apparent molecular weight, or, in the case of HA, a molecule made of repeating disaccharide units) which is differentially present in a sample taken from patients having cancer, for example, as compared to a comparable sample taken from control subjects (*e.g.*, a person with a negative diagnosis, normal or healthy subject).

The phrase "differentially present" refers to differences in the quantity and/or the frequency of a marker present in a sample taken from patients having for example, cancer, as compared to a control subject. For example, a marker can be a polypeptide which is present at an elevated level or at a decreased level in samples of patients with head and neck squamous cell carcinoma (HNSCC) compared to samples of control subjects. Alternatively, a marker can be a polypeptide which is detected at a higher frequency or at a lower frequency in samples of patients compared to samples of control subjects. A marker can be differentially present in terms of quantity, frequency or both.

A marker, compound, composition or substance is differentially present between the two samples if the amount of the marker, compound, composition or substance in one sample is statistically significantly different from the amount of the marker, compound, composition or substance in the other sample. For example, a compound is differentially present between the two samples if it is present at least about 120%, at least about 130%, at least about 150%, at least about 180%, at least about 200%, at least about 300%, at least about 500%, at least about 700%, at least about 900%, or at least about 1000% greater than it is present in the other sample, or if it is detectable in one sample and not detectable in the other.

Alternatively or additionally, a marker, compound, composition or substance is differentially present between the two sets of samples if the frequency of detecting the polypeptide in samples of patients is statistically significantly higher or lower than in the control samples. For example, a biomarker is differentially present between the two sets of samples if it is detected at least about 120%, at least about 130%, at least about 150%, at least about 180%, at least about 200%, at least about 300%, at least about 500%, at least about 700%, at least about 900%, or at least about 1000% more frequently or less frequently observed in one set of samples than the other set of samples. These exemplary values notwithstanding, it is expected that a skilled practitioner can determine cut-off points, etc. that represent a statistically significant difference to determine whether the marker is differentially present.

"Diagnostic" means identifying the presence or nature of a pathologic condition and includes identifying patients who are at risk of developing cancer. Diagnostic methods differ in their sensitivity and specificity. The "sensitivity" of a diagnostic assay is the percentage of diseased individuals who test positive (percent of "true positives"). Diseased individuals not detected by the assay are "false negatives." Subjects who are not diseased and who test negative in the assay, are termed "true negatives." The "specificity" of a diagnostic assay is 1 minus the false positive rate, where the "false positive" rate is defined as the proportion of those without the disease who test positive. While a particular diagnostic method may not provide a definitive diagnosis of a condition, it suffices if the method provides a positive indication that aids in diagnosis.

The terms "detection", "detecting" and the like, can be used in the context of detecting biomarkers, or of detecting cancers like HNSCC (*e.g.* when positive assay results are obtained). In the latter context, "detecting" and "diagnosing" are considered synonymous.

A "test amount" of a marker refers to an amount of a marker present in a sample being tested. A test amount can be either in absolute amount (*e.g.*, ng/mL) or a relative amount (*e.g.*, relative intensity of signals).

A "diagnostic amount" of a marker refers to an amount of a marker in a subject's sample that is consistent with a diagnosis of cancer or a relative amount of tumor load (*e.g.,* relative intensity of signals).

A "control amount" of a marker can be any amount or a range of amount which is to be compared against a test amount of a marker. For example, a control amount of a marker can be the amount of a marker in a person without cancer. A control amount can be either in absolute amount or a relative amount (*e.g.*, relative intensity of signals).

The terms "polypeptide," "peptide," and "protein" are used interchangeably herein to refer to a polymer of amino acid residues, in particular, of naturally-occurring amino acids. The terms apply to amino acid polymers in which one or more amino acid residue is an analog or mimetic of a corresponding naturally-occurring amino acid, as well as to naturally-occurring amino acid polymers. Polypeptides can be modified, *e.g.,* by the addition of carbohydrate residues to form glycoproteins. The terms "polypeptide," "peptide," and "protein" include glycoproteins, as well as non-glycoproteins.

"Detectable moiety" or a "label" refers to a composition detectable by spectroscopic, photochemical, biochemical, immunochemical, or chemical means. For example, useful labels include 32p, 35S, fluorescent dyes, electron-dense reagents, enzymes (*e.g.*, as commonly used in an ELISA), biotin-streptavidin, dioxigenin, haptens and proteins for which antisera or monoclonal antibodies are available, or nucleic acid molecules with a sequence complementary to a target. The detectable moiety often generates a measurable signal, such as a radioactive, chromogenic, or fluorescent signal, that can be used to quantify the amount of bound detectable moiety in a sample. Quantitation of the signal is achieved by, *e.g.,* scintillation counting, densitometry, or flow cytometry.

"Antibody" refers to a polypeptide ligand substantially encoded by an immunoglobulin gene or immunoglobulin genes, or fragments thereof, which specifically binds and recognizes an epitope (*e.g.,* an antigen). The recognized immunoglobulin genes include the kappa and lambda light chain constant region genes, the alpha, gamma, delta, epsilon and mu heavy chain constant region genes, and the myriad immunoglobulin variable region genes. Antibodies exist, *e.g.,* as intact immunoglobulins or as a number of well characterized fragments produced by digestion with various peptidases. This includes, *e.g.,* Fab' and F(ab)'2 fragments. The term "antibody," as used herein, also includes antibody fragments either produced by the modification of whole antibodies or those synthesized *de novo* using recombinant DNA methodologies. It also includes polyclonal antibodies, monoclonal antibodies, chimeric antibodies, humanized antibodies, or single chain antibodies. "Fc" portion of an antibody refers to that portion of an immunoglobulin heavy chain that comprises one or more heavy chain constant region domains but does not include the heavy chain variable region.

By "binding assay" is meant a biochemical assay wherein the biomarkers are detected by binding to an agent, such as an antibody, through which the detection process is carried out. The detection process can involve radioactive or fluorescent labels, and the like. The assay can involve immobilization of the biomarker, or can take place in solution.

"Immunoassay" is an assay that uses an antibody to specifically bind an antigen (*e.g.,* a marker). The immunoassay is characterized by the use of specific binding properties of a particular antibody to isolate, target, and/or quantify the antigen.

The phrase "specifically (or selectively) binds" to an antibody or "specifically (or selectively) immunoreactive with," when referring to a protein or peptide, refers to a binding reaction that is determinative of the presence of the protein in a heterogeneous population of proteins and other biologics. Thus, under designated immunoassay conditions, the specified antibodies bind to a particular protein at least two times the background and do not substantially bind in a significant amount to other proteins present in the sample. Specific binding to an antibody under such conditions can require an antibody that is selected for its specificity for a particular protein. A variety of immunoassay formats can be used to select antibodies specifically immunoreactive with a particular protein. For example, solid-phase ELISA immunoassays are routinely used to select antibodies specifically immunoreactive with a protein (see, *e.g.,* Harlow & Lane, Antibodies, A Laboratory Manual (1988), for a description of immunoassay formats and conditions that can be used to determine specific immunoreactivity).

"Sample" as used herein can include polynucleotides, polypeptides, peptides, antibodies fragments and derivatives thereof. A "sample" can be or can come from a bodily fluid; a soluble fraction of a cell preparation, or media in which cells were grown; a chromosome, an organelle, or membrane isolated or extracted from a cell; genomic DNA, RNA, or cDNA, polypeptides, or peptides in solution or bound to a substrate; a cell; a tissue; a tissue print; a fingerprint, saliva, blood, skin or hair; fragments and derivatives thereof.

By "at risk of' is meant an increased risk of, compared to a normal subject, or-compared to a control group, *e.g.* a patient population. Thus a subject "at risk of' developing cancer is at increased risk compared to a normal subject or population, and a subject "at risk of' a recurrence of cancer can be considered at increased risk of having a recurrence as compared to the risk of a recurrence among all treated patients.

"Increased risk" or "elevated risk" mean any statistically significant increase in the probability, *e.g.,* that the subject will develop cancer, or a recurrence thereof. The risk is preferably increased by at least 10%, more preferably at least 20%, and even more preferably at least 50% over the control group with which the comparison is being made.

The term "prognosis" means a prediction about the likely course of disease or disease progression, particularly with respect to likelihood of disease remission, disease relapse, tumor recurrence, metastasis, and death. "Good prognosis" refers to a likelihood that a patient afflicted with cancer, such as head and neck squamous cell carcinoma, will remain disease-free (*i.e.,* cancer-free). "Poor prognosis" refers to a likelihood a patient will have a relapse or recurrence of the underlying cancer or tumor, metastasis, or death. Cancer patients classified as having a "good outcome" remain free of the underlying cancer or tumor. In contrast, "bad outcome" cancer patients experience disease relapse, tumor recurrence, metastasis, or death. In particular examples, the time frame for assessing prognosis and outcome is, for example, less than one year, one, two, three, four, five, six, seven, eight, nine, ten, fifteen, twenty or more years. As used herein, the relevant time for assessing prognosis or disease-free survival time begins with the surgical removal of the tumor or suppression, mitigation, or inhibition of tumor growth. Thus, for example, in particular examples, a "good prognosis" refers to the likelihood that a head and neck squamous cell carcinoma patient will remain free of the underlying cancer or tumor for a period of at least five, more particularly, a period of at least ten years. In further examples, a "bad prognosis" refers to the likelihood that a head and neck squamous cell carcinoma patient will experience disease relapse, tumor recurrence, metastasis, or death within less than five years, more particularly less than ten years. Time frames for assessing prognosis and outcome provided above are illustrative and are not intended to be limiting.

The term "treatment" refers to the medical management of a patient with the intent to cure, ameliorate, stabilize, or prevent a disease, pathological condition, or disorder. This term includes active treatment, that is, treatment directed specifically toward the improvement of a disease, pathological condition, or disorder, and also includes causal treatment, that is, treatment directed toward removal of the cause of the associated disease, pathological condition, or disorder. In addition, this term includes palliative treatment, that is, treatment designed for the relief of symptoms rather than the curing of the disease, pathological condition, or disorder; preventative treatment, that is, treatment directed to minimizing or partially or completely inhibiting the development of the associated disease, pathological condition, or disorder; and supportive treatment, that is, treatment employed to supplement another specific therapy directed toward the improvement of the associated disease, pathological condition, or disorder.

The term "lifestyle counseling" or "risk factor management counseling" refers to professional counseling administered to a patient regarding the patient's lifestyle. For example, the patient can receive counseling for addictions, such as smoking, drinking, or drug use, or can receive counseling concerning sexual behavior, such as counseling regarding lower-risk sexual behavior, condom use, etc. "Lifestyle counseling" can also refer to diet modification or stress management. Lifestyle counseling is administered by a professional, and can include one or more treatment sessions, literature, professional videos, etc.

The phrase "similar to" as in individuals in the populations have an age similar to that of the subject means that the average age of the population is with 10 year, e.g., within 5 years of the subject. Also, the phrase "similar to" as in individuals in the populations have a race similar to the race of the subject means the subject is or has at least one parent that is the same race as the majority of the individuals in the population. The phrase "similar to" as in individuals in the populations have a history of alcohol consumption, history of tobacco use similar to the subject means the average years of alcohol or smoking are within 10 years, e.g., 5 years, of the subject.

### Methods

Upper aerodigestive tract (UADT) mucosa progresses through a premalignant phase dysplasia, prior to development of frank malignancy. Identifying lesions in this stage is desirable because dysplasia is reversible (Pindborg, A follow up study of sixty one oral dysplastic precancerous lesions in Indian villagers. Oral Surg Oral Med Oral Pathol (1977) 43:383-90) and can regress spontaneously or with tobacco cessation (Larsson, et al. Reversibility of snuff dippers' lesions in Swedish moist snuff users: a clinical and histologic follow-up study. Oral Pathol Med. (1991) 20(6):258-64; Grizzle, et al. The biology of incipiend, pre-invasive or intraepithelial neoplasia. Cancer Biomark (2010) 9:21-39). Unfortunately, dysplasia is only sometimes visible and often mimics findings that are also seen with benign inflammation. Frequently dysplasia remains occult until further progression resulting in late stage diagnosis (Poh, et al. Direct fluorescence visualization of clinically occult high-risk oral premalignant disease using a simple hand-held device. Head Neck (2007) 29(1):71-6).

CD44, a cell surface transmembrane glycoprotein involved in cell proliferation, cell migration, and tumor initiation (Screaton et al. Proc Natl Acad Sci USA (1992) 89:12160-4; Ponta et al. Nature Rev Mol Cell Biol (2003) 4:33-45; Perez et al. Oral Oncol 2012; Prince et al. Proc Natl Acad Sci USA (2007) 104:973) is overexpressed in premalignant lesions (Hirvikoski et al. Virchows Arch. (1999) 43437-44; Ioachim et al. Histol Histopathol (1999) 14:1113-8). As mucosal changes progress from normal to severe dysplasia, CD44 expression advances from the basal layers to involve all layers of the epithelium (Hirvikoski et al. Virchows Arch. (1999) 43437-44; Ioachim et al. Histol Histopathol (1999) 14:1113-8). Furthermore, CD44 is released by proteinases into a soluble form (solCD44) that is detectable in body fluids (Kajita et al. J. Cell Biol (2001) 153:893-904). Total protein is also an effective tumor marker (Franzmann et al. Head & neck (2012) 34:687-95; Pereira et al. Cancer Biomark (2011)10:241-9; Franzmann et al. Cancer Epidemiol Biomarkers Prev (2007) 16:1348-55).

Disclosed herein is a noninvasive diagnostic test that measures markers in a bodily fluid sample using a soluble CD44 (solCD44) immunoassay and a total protein assay. Both solCD44 and total protein levels are higher in HNSCC compared to controls, associated with poor prognosis, and are elevated before cancers are visible by standard oral exam. Furthermore, it has been shown that cancers in other locations such as lung and bladder can also be detected (Example 1).

Monitoring solCD44 and total protein levels over time allows clinicians to pinpoint those patients in need of subspecialist exam, molecular imaging and biopsy. The data in patients who progressed from no evidence of disease to confirmed premalignancy or cancer (Example 1) show that solCD44 and protein levels are increased above normal, in some cases, more than 2 years before malignancy or premalignancy is clinically visible. This lead time can facilitate smoking cessation interventions since premalignancy is a reversible state (Pindborg, et al. A follow-up study of sixty-one oral dysplastic precancerous lesions in Indian villagers. Oral Surg Oral Med Oral Pathol. (1977) 43:383-90) and can regress with tobacco cessation (Larsson, et al. Reversibility of snuff dippers' lesion in Swedish moist snuff users: a clinical and histologic follow-up study. Oral Pathol Med (1991) 20(6):258-64).

Disclosed herein is a method of determining a risk of cancer in a subject that includes providing a sample of bodily fluid from the subject; measuring a level of solCD44 in the sample, i.e., a test amount of solCD44; measuring a level of total protein in the sample, i.e., a test amount of total protein; providing a reference level of solCD44 and a reference level of total protein, wherein the reference levels of solCD44 and total protein are determined by using solCD44 and total protein levels from populations of healthy individuals and individuals with the cancer, and wherein the reference levels of solCD44 and total protein delimit different, statistically significant risks for the cancer; and determining the risk of the cancer in the subject by determining whether the test amount of solCD44 and the test amount of total protein are above or below the reference levels of solCD44 and total protein. That is, the reference levels of solCD44 and total protein are used as thresholds between statistically significant risk such that test amounts of solCD44 and total protein above these reference levels indicates a statistically significant risk (e.g., twice a likely, ten times as likely, etc.) the subject has cancer as opposed to when a subject's test levels of solCD44 and total protein are below the reference levels.

Also disclosed is a method of determining a cancer prognosis in a subject that includes providing a sample of a bodily fluid of the subject; measuring a test amount of solCD44 in the sample; measuring a test amount of total protein in the sample; providing a reference level of solCD44 and a reference level of total protein, wherein the reference levels of solCD44 and total protein are determined by using solCD44 and total protein levels from populations of individuals with a good prognosis and individuals with a poor prognosis for the cancer, and wherein the reference levels of solCD44 and total protein delimit different, statistically significant prognosis for the cancer; determining the cancer prognosis in the subject by determining whether the test amount of solCD44 and the test amount of total protein are above or below the reference levels of solCD44 and total protein. Here, the reference levels of solCD44 and total protein are used as thresholds between statistically significant prognosis (good vs. poor) such that test amounts of solCD44 and total protein above these reference levels indicates a statistically significant prognosis (e.g., twice a likely, ten times as likely, etc.) the subject has a poor prognosis as opposed to when a subject's test levels of solCD44 and total protein are below the reference levels.

Also disclosed is a method of determining the effectiveness of a cancer treatment in a subject being treated for cancer that includes providing a sample of a bodily fluid of the subject; measuring a test amount of solCD44 in the sample; measuring a test amount of total protein in the sample; providing a reference level of solCD44 and a reference level of total protein, wherein the reference levels of solCD44 and total protein are determined by using solCD44 and total protein levels from populations of healthy individuals and individuals with the cancer, and wherein the reference levels of solCD44 and total protein delimit different, statistically significant outcomes for the cancer; determining the effectiveness of the cancer treatment by determining whether the test amount of solCD44 and the test amount of total protein are above or below the reference levels of solCD44 and total protein. Here, the reference levels of solCD44 and total protein are used as thresholds between statistically significant outcomes for the cancer such that test amounts of solCD44 and total protein above these reference levels indicates a statistically significant outcome (*e.g.,* twice as likely, ten timex as likely, etc.) the subject has a given outcome (*e.g.,* remission) as opposed to when a subject's test levels of solCD44 and total protein are below the reference levels.

The methods described herein correlate solCD44 and total protein levels in a subject with cancer. In particular, the methods disclosed herein are able to determine the risk of cancer in a subject. The subject can be in a "high risk" category, meaning that they have one or more risk factors that have been found to correlate with cancer. These "high risk" categories can include, but are not limited to, the subject's age, race, smoking status, alcohol consumption, history of cancer, and/or positive result of a human papilloma virus (HPV) assay. These risk factors can be used, along with the test amount of solCD44 and total protein to determine a subject's likelihood of developing malignancy, or pre-malignancy. "Pre-malignancy" is defined as tissue that is not yet malignant but is poised to become malignant. Examples of premalignant growths include polyps in the colon, actinic keratosis of the skin, dysplasia of the cervix, metaplasia of the lung, and leukoplakia (white patches in the mouth). In some instances, premalignancy does not necessarily show any clinical symptoms at all.

The subject may not have been diagnosed as being pre-malignant or malignant, or may not have been examined prior to the assay disclosed herein. The subject may have no clinically visible signs of malignancy or premalignancy.

After a diagnosis of a level of solCD44 and total protein above a threshold level, the patient can be referred to a specialist for further analysis and treatment. The subject can then be exposed to surgery, radiation, or chemotherapy, or a combination thereof, based on the comparison of the score with prior values, wherein the subject was not previously exposed to surgery, radiation, or chemotherapy, or a combination thereof. The subject can also be provided with risk factor management, or lifestyle, counseling. For example, the subject can be counseled regarding smoking, drinking, and other at-risk behaviors. The subject, for example, can be enrolled in a smoking cessation program.

The subject can also be asked to return for follow-up measurements and assays. The subject can be re-tested for solCD44 and total protein levels every week, month, 6 months, year, or 5 years, or any amount in between. This can be done in conjunction with visits to a specializing physician and risk factor management counseling, as described herein.

"Determining a risk of a cancer in a subject" is intended to mean that overexpression of the combination of biomarkers is associated with an increased likelihood of a tumor, metastasis, or death. For example, "risk of cancer in a subject" can refer to an increased likelihood of cancer or tumor, metastasis, or death within one year, five years, ten years, or more, or any amount of time in-between. solCD44 or total protein can be overexpressed by 10%, 20%, 30%, 40%, 50%, 60%, 70%, 80%, 90%, 100%, 200%, 300%, 400%, 500%, or greater as compared to a control.

### CD44

solCD44 is elevated in the majority of head and neck squamous cell carcinoma (HNSCC) cases, and it distinguishes cancer from benign disease with high specificity. CD44 is expressed on the basal surface of normal upper aerodigestive tract epithelium. CD44 expression increases and involves all layers in epithelium, with histologic dysplastic changes in 90% of cases. CD44v promotes tumorigenesis, as it interacts with matrix metalloproteinase. MMP type 1 cleaves CD44 to soluble form (solCD44). It has been shown that oral rinses for HNSCC patients had solCD44 levels that were greater than 7 times higher than normal controls. 80% of HNSCC had elevated salivary solCD44, while none of the controls did.

A CD44-based screening test has been discovered which is rooted on the molecule's known role in tumor biology. CD44 is overexpressed as a result of tobacco induced genetic damage and is required for tumor initiation. Most HNSCC occurs in smokers, even if the tumors are HPV positive (Lin, et al. Long-term prognosis and risk factors among patients with HPV-associated oropharyngeal squamous cell carcinoma. Cancer (2013). Tobacco smoke induces damage by creating DNA adducts and oxidative damage which lead to mutations (Pfeifer, et al. Tobacco smoke carcinogens, DNA damage and p53 mutations in smoking-associated cancers. Oncogene (2002) 21:7435-51) and DNA double strand breaks (Werbrouk, et al. Single-nucleotide polymorphisms in DNA double-strand break repair genes: association with head and neck cancer and interaction with tobacco use and alcohol consumption. Mutation Res (2008) 656:74-81), respectively. When DNA breaks are not correctly repaired, genetic aberrations such as gene amplification occurs (Mondello, et al. Gene amplification, radiation sensitivity and DNA double -strand breaks. Mutation Res 2010; Miller, et al. Genomic amplification of MET with boundaries within fragile site FRA7G and upregulation of MET pathways in esophageal adenocarcinoma. Oncogene (2006) 25:409-18; Jarvinen, et al. High-resolution copy number and gene expression microarray analysis of head and neck squamous cell carcinoma cell lines of tongue and larynx. Genes, Chromosomes & Cancer (2008) 47:500-9) leading to overexpression of CD44 and other gene products (Miller, et al. Genomic amplification of MET with boundaries within fragile site FRA7G and upregulation of MET pathways in esophageal adenocarcinoma. Oncogene (2006) 25:409-18; Liu, *et al.* (1978). CD44 represents a family of transmembrane glycoproteins with a common domain and a variable region of alternatively spliced exons (exons 5-14) (Screaton GR, et al. Genomic structure of DNA encoding the lymphocyte homing receptor CD44 reveals at least 12 alternatively spliced exons. Proc Natl Acad Sci USA (1992) 89:12160-4). CD44 isoforms interact with many other molecules including extracellular matrix components (hyaluronic acid) (HA), membrane proteins (EGFR, HER2), cytoskeletal components (ezrin, radixin, moesin and merlin) and intranuclear proteins (STAT 3) resulting in oncogenic signaling (Ponta, et al. CD44: from adhesion molecules to signaling regulators. Nature Rev Mol Cell Biol (2003) 4:33-45; Morrison *et al.* (1998); Lokeshwar, et al. Ankyrin-binding domain of CD44 is required for the expression of hyaluronic acid-mediated adhesion function. J Cell Biol (1994) 126:1099-1109; Lee, et al. Acetylation and activation of STAT3 mediated by nuclear translocation of CD44. J Cell Biol. (2009) 185:949-57). This signaling inhibits apoptosis (Perez, et al. CD44 interacts with EGFR and promotes head and neck squamous cell carcinoma initiation and progression. Oral Oncol. (2013) 4:306-13; Lakshman, et al. CD44 promotes resistance to apoptosis in human colon cancer cells. Exp Mol Pathol (2004) 77:18-25) promoting further genetic damage and malignant transformation.

CD44 is not merely a byproduct of tumorigenesis, but a driver of tumor initiation that can be easily and noninvasively detected by an oral rinse test. Tumor cells that are CD44 positive (CD44+) have tumor initiating capacity (Prince ME, et al. Identification of a subpopulation of cells with cancer stem cell properties in head and neck squamous cell carcinoma. Proc Natl Acad Sci USA (2007) 104:973-8). CD44 negative (CD44-) tumor cells produce tumors in only 1 out of 40 implantations, while CD44+ cells produced tumors in 20 of 30 implantations with injection of only a few thousand cells (Prince, et al. Identification of a subpopulation of cells with cancer stem cell properties in head and neck squamous cell carcinoma. Proc Natl Acad Sci USA (2007) 104:973-8).

CD44 is a receptor for hyaluronic acid and can also interact with other ligands, such as osteopontin, collagens, and MMPs. CD44 is a multi-structural and multi-functional cell surface molecule involved in cell proliferation, cell differentiation, cell migration, angiogenesis, presentation of cytokines, chemokines, and growth factors to the corresponding receptors, and docking of proteases at the cell membrane, as well as in signaling for cell survival. All these biological properties are essential to the physiological activities of normal cells, but they are also associated with the pathologic activities of cancer cells. CD44 expression can be measured easily in bodily fluids because proteases such as Membrane-Type 1 MMP (MT1- MMP) cleave CD44 to its soluble form (solCD44) (Kajita, et al. Membrane-type 1 matrix metalloproteinase cleaves CD44 and promotes cell migration. J Cell Biol (2001) 153:893-904).

The disclosed assays involve the detection of one or more biomarkers, such as CD44 (e.g., soluble CD44 (solCD44)), in a sample from the subject. U.S. Patent No. 8,088,591 by Franzmann et al. describes biomarkers that can be used to diagnose and monitor HNSCC in a subject.

CD44 is expressed in a large number of mammalian cell types. The standard isoform, designated CD44s, comprising exons 1-5 and 16-20 is expressed in most cell types. CD44 splice variants containing variable exons are designated CD44v. Some epithelial cells also express a larger isoform (CD44E), which includes exons v8-10. CD44 proteins are also released in soluble form (solCD44) via proteases (Kajita, et al. J Cell Biol (2001) 153:893-904) and are detectable in normal circulation (Naor, et al. Adv Cancer Res (1997) 71:241-319; Guo, et al. Cancer Res (1994) 54:422-6; Martin, et al. Int J Cancer (1997) 74:443-5; Ristamaki, et al. Blood (1997) 90:4039-45;Yamane, et al. Oncology (1999) 56:232-8; Scott, et al. Cancer Epidemiol Biomarkers Prev (2000) 9:1211-4; Van Hal, et al. Clin Cancer Res (1999) 5:3534-4). Circulating levels of solCD44 correlate with metastases in some tumors (Ristamaki et al. Blood (1997) 90:4039-45; Yamane et al. Oncology (1999) 56:232-8).
solCD44 can be measured by immunoassay. Immunoassays include, but are not limited to, ELISA, MELISA, CEDIA, immunoscreening, lateral flow test (lateral flow assay), magnetic immunoassay, radioimmunoassay, or Surround Optical Fiber Immunoassay (SOFIA). Further examples include Enzyme Linked Immunosorbent Assay (ELISA) or Lateral Flow Assay.

### Total Protein

Total Protein (TP), as it is referred to herein, is the amount of all protein present in a sample, for example, in an oral rinse. The simplest and most direct assay method for proteins in solution is to measure the absorbance at 280nm (UV range). Amino acids containing aromatic side chains (*i.e*., tyrosine, tryptophan and phenylalanine) exhibit strong UV-light absorption. Consequently, proteins and peptides absorb UV-light in proportion to their aromatic amino acid content and total concentration. Another method, traditionally used in amino acid analysis by HPLC, is to label all primary amines (*i.e.,* N-terminus and side-chain of lysine residues) with a colored or fluorescent dye such as ninhydrin or o-phthaldialdehyde (OPA). Several colorimetric, reagent-based protein assay techniques are also known. Protein is added to the reagent, producing a color change in proportion to the amount added. Proteins concentration is determined by reference to a standard curve consisting of known concentrations of a purified reference protein.

Examples of total protein assays include the Bradford assay, Lowry assay, modified Lowry, and Pierce BCA Protein Assay. The Lowry assay is a biochemical assay for determining the total level of protein in a solution. The total protein concentration is exhibited by a color change of the sample solution in proportion to protein concentration, which can then be measured using colorimetric techniques. There are also modifications to the Lowry Assay, which can be used with the methods disclosed herein. One example is found in Peterson et al. (Anal Biochem (1977) 83(2):346-356).

### Statistical Analysis

Reference levels of solCD44 and of total protein, as disclosed herein, can be determined by using solCD44 and total protein levels from populations of healthy individuals and individuals with cancer, wherein the reference levels of solCD44 and total protein delimit different, statistically significant risks for the cancer. These reference levels can be subjected to statistical analysis, such as logistic regression calculations. Multivariate analysis can be used in the statistical analysis as well. Methods of using logistic regression calculations with multivariate analysis are known in the art, such as those set forth in U.S. Patent 6,110,109.

Reference levels of solCD44 and total protein from healthy individuals, as well as those with cancer, can be subjected to regression analysis. It is noted that this analysis can be done for individuals with a certain type of cancer, such as an HNSCC, for example. These scores can be analyzed to determine statistically significant delimitations of cancer risk. Once these score are obtained, they can be applied to the methods disclosed herein to determine the risk of cancer in the subject by determining whether the test amount of solCD44 and the test amount of total protein in a patient are above or below the reference levels of solCD44 and total protein. An individual patient's risk level can thus be assessed based on this comparison.

Methods of calculating logistic regression, as well as multivariate analysis methods, are disclosed in Gareth James et al., (2013). An Introduction to Statistical Learning. Springer. p. 6; Harrell, Frank E. (2001). Regression Modeling Strategies. Springer-Verlag. ISBN 0-387-95232-2; Hosmer, David et al. (2000). Applied Logistic Regression (2nd ed.). Wiley. ISBN 0-471-35632-8; Menard, Scott W. (2002). Applied Logistic Regression (2nd ed.). SAGE. ISBN 978-0-7619-2208-7; Cohen, Jacob et al. (2002). Applied Multiple Regression/Correlation Analysis for the Behavioral Sciences (3rd ed.). Routledge. ISBN 978-0-8058-2223-6; and Mark et al. (2001). Multiple Regression Analysis and Mass Assessment: A Review of the Issues. The Appraisal Journal, Jan. pp. 89-109.

### Sample Collection

The sample to be used in the method of the invention is an oral saline rinse. Samples to be used in other methods disclosed herein can be obtained from any bodily fluid. For example, the bodily fluid can be selected from the group consisting of oral rinse, saliva, sputum, breath condensate, blood, blood plasma, serum, and urine. Saliva can be collected using many methods. One common method is whole saliva collection. Saliva is collected, often over a set period of time, from the anterior oral cavity, where the majority is released under resting conditions. Oral rinses involve use of a set amount of a fluid, often saline, that is manipulated in the mouth and helps release substances adherent to the lining of the oral cavity, larynx and pharynx.

The subject can be asked to abstain from eating, drinking, or smoking prior to the oral rinse sample being obtained. The subject can be asked to abstain for 10, 20, 30, 40, 50, or 60 minutes or longer, for example.

### Cancers

The method of the invention is for determining the risk of head and neck squamous cell cancer. Other methods disclosed herein can be used to detect any type of cancer, or combinations of more than one type of cancer. Examples, include, but are not limited to, Leukemia, acute leukemia, acute lymphocytic leukemia, acute myelocytic leukemia, myeloblastic, promyelocytic, myelomonocytic, monocytic, erythroleukemia, chronic leukemia, chronic myelocytic (granulocytic) leukemia, chronic lymphocytic leukemia, Polycythemia vera, Lymphoma, Hodgkin's disease, non-Hodgkin's disease, Multiple myeloma, Waldenstrom's macroglobulinemia, Heavy chain disease, Solid tumors, sarcomas and carcinomas, fibrosarcoma, myxosarcoma, liposarcoma, chondrosarcoma, osteogenic sarcoma, chordoma, angiosarcoma, endothelio sarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, menangioma, melanoma, neuroblastoma, and retinoblastoma. (For a review of such disorders, see Fishman et al., (1985, Medicine, 2d Ed., J.B. Lippincott Co., Philadelphia.)

In particular, the cancer can be selected from the group consisting of head and neck squamous cell cancer (HNSCC), lung cancer, prostate cancer, colon cancer, bladder cancer, melanoma, leukemia/lymphoma, breast cancer, and osteoscaroma.

Also disclosed herein are "pre-malignancies," which precede progression to neoplasia or cancer, in particular, where non-neoplastic cell growth consisting of hyperplasia, metaplasia, or most particularly, dysplasia has occurred (for review of such abnormal growth conditions, see Robbins and Angell, 1976, Basic Pathology, 2d Ed., W.B. Saunders Co., Philadelphia, pp. 68-79.). Hyperplasia is a form of controlled cell proliferation involving an increase in cell number in a tissue or organ, without significant alteration in structure or function. As but one example, endometrial hyperplasia often precedes endometrial cancer. Metaplasia is a form of controlled cell growth in which one type of adult or fully differentiated cell substitutes for another type of adult cell. Metaplasia can occur in epithelial or connective tissue cells. A typical metaplasia involves a somewhat disorderly metaplastic epithelium. Dysplasia is frequently a forerunner of cancer, and is found mainly in the epithelia; it is the most disorderly form of non-neoplastic cell growth, involving a loss in individual cell uniformity and in the architectural orientation of cells. Dysplastic cells often have abnormally large, deeply stained nuclei, and exhibit pleomorphism. Dysplasia characteristically occurs where there exists chronic irritation or inflammation, and is often found in the cervix, respiratory passages, oral cavity, and gall bladder.

### Patient Risk

The subject disclosed herein can be analyzed for risk factors other than the measurement of total protein and solCD44. For example, the patient risk factor can comprise alcohol use, tobacco use, or human papillomavirus (HPV) infection. The patient risk factor can also include a laboratory result regarding another cancer marker.

Traditionally, 80%-90% of HNSCC have been attributed to tobacco and alcohol use (Sturgis, et al. Cancer (2007) 110:1429-35). The risk for developing HNSCC for cigarette smokers is 5- to 25-fold greater than that for nonsmokers, with the risk increasing in a dose-response fashion with frequency, duration, and extent of smoking (Marur et al. Mayo Clin Proc. (2008) 83:489-501; Sturgis, et al. Cancer (2007) 110:1429-35; Ragin, et al. J Dent Res (2007) 86:104-14; Curado et al. Curr Opin Oncol. (2009) 21:194-200). The risk substantially decreases with time from smoking cessation, although this risk never reaches the level of a never-smoker (Schlect, et al. Epidemiology (1999) 10:412-18; Bosetti, et al. Am J Epidemiol (2008) 167:468-73). One Italian study found that the cumulative risk at 75 years of age for all upper aerodigestive tract cancers was 6.3% for men who continued to smoke any type of tobacco, 3.1% for men who stopped smoking at around 50 years of age, 1.2% for men who stopped smoking at around 30 years of age, and 0.8% among lifelong nonsmokers (Bosetti, et al. Am J Epidemiol. (2008) 167:468-73). Although active tobacco smoking is the major risk factor for HNSCC, involuntary or secondhand smoking has also been associated with an increased cancer risk. In an international pooled analysis, long exposure to involuntary smoking, described as occurring over a period of more than 15 years, both at home and work, was associated with an increased risk of HNSCC, particularly pharyngeal and laryngeal cancers (Lee et al. Cancer Epidemiol Biomarkers Prev (2008) 17:1974-81).

Alcohol has also been shown to synergistically increase the HNSCC risk attributable to tobacco use (Ragin et al. J Dent Res (2007) 86:104-14). Heavy alcohol consumption alone is an independent risk factor for HNSCC. High-frequency use, defined as three or more drinks per day, was independently associated with an increased risk of cancers of the oropharynx, hypopharynx, and larynx (Hashibe et al. J Natl Cancer Inst (2007) 99:777-89).

Other risk factors that have been associated with HNSCC include dietary patterns, viruses, and occupational exposures. Numerous studies have associated HNSCC with vitamin A deficiency, whereas other studies describe an inverse association with high fruit and vegetable intake (Marur et al. Mayo Clin Proc. (2008) 83:489-501; Curado et al. Curr Opin Oncol. (2009) 21:194-200). Viruses that have been implicated include Epstein-Barr virus (EBV) and human papillomavirus (HPV), and occupational exposures such as chromium, nickel, and radium have also been linked particularly to sinonasal carcinomas (Marur et al. Mayo Clin Proc. (2008) 83:489-501).

### HPV

The incidence of smoking-associated HNSCC has decreased in the US since 1975, while the proportion of HNSCC that are potentially attributable to Human Papilloma Virus (HPV) has increased. Over 2,370 new cases of HPV-associated oropharyngeal squamous cell carcinoma (OPSCC) are diagnosed in women, and nearly 9,356 cases are diagnosed in men each year in the United States alone. The estimated lifetime risk of cervical HPV infection in sexually active women is up to 80%. Disclosed herein is the immunologic link between HPV and induced oropharyngeal cancer, and cervical cancer. PD-1 (programmed cell death) receptor is a common link in immune evasion of tumors. (2008, World Health Organization Section of Cancer Information, 2008; Goodman, MT, *et al.;* 2012, Lyford-Pike, S, *et al*.)

solCD44 and total protein levels combined are more effective at distinguishing HNSCC from controls than either marker alone. However, solCD44 levels can be lower in subjects with human papillomavirus (HPV) infection. In fact, bivariate analysis using solCD44 and total protein levels works best in black men, wherein HPV infection is less common. Therefore, inclusion of HPV status in a multivariate analysis can improve sensitivity and accuracy of the assay and allow for detection of HPV+ HNSCC. (Example 2).

Other biomarkers associated with HNSCC detection or prognosis may be used in combination with total protein, solCD44, and HPV detection to improve sensitivity and/or accuracy of the disclosed method. For example, solCD44 levels can vary based on age and smoking status. Examples of HNSCC risk factors and demographic factors that may be used in the multivariate analysis include tobacco exposure, alcohol exposure, race, ethnicity, dental health, gender, level of education, age, general health, family history of cancer, sexual history and socioeconomic status and using the one or more risk factors or demographic factors in the multivariate analysis to determine the combined score.

HPV infection can be determined by measuring HPV directly or indirectly. Three categories of molecular assays are currently available for detection of HPV infection in tissue and exfoliated cell samples. All are based on detection of HPV DNA and include: (1) non-amplified hybridization assays (Southern transfer hybridization, (STH), dot blot hybridization (DB) and in situ hybridization (ISH)); (2) Signal amplified hybridization assays such as hybrid capture assays; and (3) Target amplification assays, such as PCR and in situ PCR. Southern blot hybridization requires large amounts of DNA, is laborious, and is not reproducible, while in situ hybridization has only moderate sensitivity for HPV. PCR-based detection of HPV is both extremely sensitive and specific. Using this approach, the viral DNA is amplified *in vitro* by DNA polymerase to generate adequate amount of target, which is then either directly visualized on gels, or (the more specific approach) detected by specific probe using traditional hybridization methods. In practice, the sensitivity of PCR based method is about 10-100 HPV viral genomes in a background of 100 ng cellular DNA. Since PCR can be performed on very small amounts of DNA (10-100 ng), it is ideal for use on specimens with low DNA content.

### Genetic Factors

In other examples, a patient which exhibits one or more of the following predisposing factors for malignancy can be selected for the methods disclosed herein: a chromosomal translocation associated with a malignancy, familial polyposis or Gardner's syndrome (possible forerunners of colon cancer), benign monoclonal gammopathy (a possible forerunner of multiple myeloma), and a first degree kinship with persons having a cancer or precancerous disease showing a Mendelian (genetic) inheritance pattern (e.g., familial polyposis of the colon, Gardner's syndrome, hereditary exostosis, polyendocrine adenomatosis, medullary thyroid carcinoma with amyloid production and pheochromocytoma, Peutz-Jeghers syndrome, neurofibromatosis of Von Recklinghausen, retinoblastoma, carotid body tumor, cutaneous melanocarcinoma, intraocular melanocarcinoma, xeroderma pigmentosum, ataxia telangiectasia, Chediak-Higashi syndrome, albinism, Fanconi's aplastic anemia, and Bloom's syndrome; see Robbins and Angell, 1976, Basic Pathology, 2d Ed., W.B. Saunders Co., Philadelphia, pp. 112-113) etc.)

### HA and HAase

The level of hyaluronic acid (HA) and hyaluronidase (HAase) can also be measured in the sample. HA is a nonsulfated glycosaminoglycan (GAG), overexpressed in certain cancers. HA is synthesized by hyaluronan synthase on the surface of cells and is comprised of repeating disaccharide units of D-glucuronic acid and N-acetyl-D-glucosamine. It is present in body fluids, tissues, and extracellular matrix. It interacts with cell surface receptors (*e.g.*, CD44, RHAMM, etc.) and, through these interactions, regulates cell adhesion, migration, and proliferation. Depending upon the type of tumor, HA may be synthesized by stromal cells, tumor cells or both. In tumor tissues, HA supports metastasis by promoting tumor cell migration, offering protection against immune surveillance and causing a partial loss of contact-medicated inhibition of cell growth and migration. Small fragments of HA are angiogenic and have been isolated from urine of bladder cancer patients, prostate cancer tissue, and saliva from HNSCC patients. Concentrations of HA are elevated in several cancers, including colon, breast, prostate, bladder and lung. Tissue expression of HA in tumors such as colon and breast, indicates a poor prognosis.

**Table 1: Comparison of CD44 and Total Protein Levels in Patients with and without Cancer History**

| | | R01 Controls | | | t-test P |
|---|---|---|---|---|---|
| | | All | Cancer History | | |
| | | | No | Yes | |
| | N | 150 | 134 | 16 | |
| | | | | | |
| CD44 | **Mean** | **2.87** | **2.75** | **3.86** | 0.021 |
| | StdErr | 0.15 | 0.15 | 0.59 | |
| | StdDev | 1.83 | 1.73 | 2.34 | |
| | Median | 2.27 | 2.21 | 3.00 | |
| | Min | 0.17 | 0.17 | 1.96 | |
| | Max | 11.58 | 11.58 | 9.72 | |
| | | | | | |
| Log2 CD44 | **Mean** | **1.28** | **1.23** | **1.75** | 0.017 |
| | StdErr | 0.07 | 0.07 | 0.18 | |
| | StdDev | 0.83 | 0.83 | 0.74 | |
| | Median | 1.18 | 1.15 | 1.58 | |
| | Min | -2.56 | -2.56 | 0.97 | |
| | Max | 3.53 | 3.53 | 3.28 | |
| | | | | | |
| Protein | **Mean** | **0.76** | **0.74** | **0.96** | 0.039 |
| | StdErr | 0.03 | 0.03 | 0.10 | |
| | StdDev | 0.39 | 0.39 | 0.42 | |
| | Median | 0.66 | 0.64 | 1.03 | |
| | Min | 0.05 | 0.05 | 0.29 | |
| | Max | 1.92 | 1.92 | 1.56 | |

**Table 2: CD44 and Protein Levels in Patients with Various Past Cancers**

| Past Cancer History | CD44 | Log2 CD44 | Protein |
|---|---|---|---|
| colon | 8.195 | 3.035 | 1.339 |
| prostate | 6.480 | 2.696 | 1.269 |
| prostate | 2.145 | 1.101 | 0.559 |
| prostate | 3.785 | 1.920 | 1.255 |
| melanoma | 9.715 | 3.280 | 1.175 |
| bladder | 2.920 | 1.546 | 0.716 |
| prostate | 3.810 | 1.930 | 1.026 |
| lymphoma/leukemia | 4.305 | 2.106 | 1.384 |
| prostate/melanoma | 4.375 | 2.129 | 1.558 |
| prostate | 2.165 | 1.114 | 0.301 |
| osteosarcoma | 2.295 | 1.198 | 1.463 |
| colon | 3.080 | 1.623 | 1.028 |
| prostate | 2.265 | 1.180 | 0.562 |
| breast | 2.110 | 1.077 | 0.852 |
| prostate | 1.963 | 0.973 | 0.512 |
| bladder | 2.185 | 1.128 | 0.293 |

HAase is an endoglycosidase that degrades HA into small angiogenic HA fragments. HA and HA fragments stimulate endothelial cell proliferation, adhesion and migration by activating the focal adhesion kinase and MAP kinase pathways. HAase alters the expression of CD44 isoforms and is associated with increased tumor cell cycling. Of the 6 human HAases encoded by different genes, three are characterized at the protein level.

### Kits

Also disclosed herein are kits that include a saline solution; a cup for receiving an oral saline rinse; at least one antibody that specifically binds CD44; a reagent for determining total protein concentration; and reference levels for solCD44 and total protein, wherein the reference levels of solCD44 and total protein are determined by using solCD44 and total protein levels from populations of healthy individuals and individuals with the cancer, and wherein the reference levels of solCD44 and total protein delimit different, statistically significant risks for the cancer.

### EXAMPLES

The following examples are set forth below to illustrate the methods, compositions, and results according to the disclosed subject matter. These examples are not intended to be inclusive of all aspects of the subject matter disclosed herein, but rather to illustrate representative methods, compositions, and results. These examples are not intended to exclude equivalents and variations of the present invention, which are apparent to one skilled in the art.

### Example 1: CD44 and Total Protein Oral Rinse Test

The solCD44/total protein oral rinse test is a powerful tool for identifying early and aggressive HNSCC. In a trial of 150 cancer cases and 150 clinic-based, frequency-matched controls, it was noted that the oral rinse CD44/total protein test distinguished HNSCC cases from controls with nearly 90% accuracy in the subset of black males. High solCD44 levels in cases were associated with poor progression free survival (PFS) and overall survival (OS) independent of protein level, disease type (oropharynx v. oral cavity), tumor stage, and other covariates.

The specificity of the solCD44/protein oral rinse test is better than anticipated in a community-based cohort. There are high rates of smoking and several tobacco-associated cancer clusters in South Florida, including a socio-economically disadvantaged city located in the Miami-Dade county ("Community Cohort) (Dietz NA, et al. Toward the identification of communities with increased tobacco-associated cancer burden: Application of spatial modeling techniques. J Carcinog 2011; 10:22), a minority rich, economically disadvantaged neighborhood in north-central Miami-Dade County. Cancer rates in the Community Cohort clusters relative to the rest of South Florida were all highly significant: lung relative risk (RR)=1.4; p<0.0001; oral cavity RR=1.5; p<0.01; and cervical RR=2.6; p<0.0001 (Dietz NA, et al. Toward the identification of communities with increased tobacco-associated cancer burden: Application of spatial modeling techniques. J Carcinog 2011; 10:22). A Community Cohort HNSCC early detection and prevention program was developed. CD44 and protein levels were assessed, and their variability over time in this high-risk population, and it was determined if smoking cessation resulted in decreased levels. It was also evaluated if the oral rinse test was acceptable. The specificity of the oral rinse test was validated in subjects from Community Cohort and it was found that the test distinguishes black male HNSCC cases (enrolled in the clinic-based study) from Community Cohort controls with 100% sensitivity and 92% specificity. Of the 7 "false positives", one has developed confirmed lung cancer and another has suspected oral premalignancy. The oral rinse test is well-accepted. Furthermore, mean solCD44 levels decreased by 9% in subjects who returned for the annual visit, showing that cancer risk can be decreasing in the screened cohort. Thus this program can significantly and positively impact patients at risk for HNSCC.

### Data

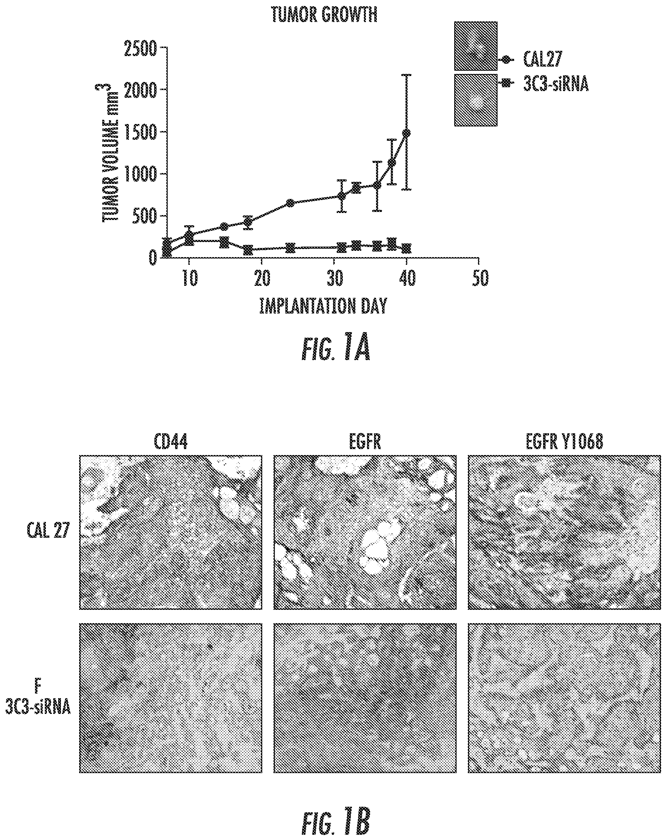

*CD44 is involved in tumor initiation and progression:* CD44 is a transmembrane protein expressed on the cell surface and involved in tumor initiation. In normal mucosa, CD44 staining is confined to the basal and parabasal layers but increases to involve all layers with increasing dysplasia and invasive cancer. Poorly differentiated tumors show focal CD44 staining surrounding blood vessels and at the periphery in areas of cancer expansion (Germani, R, et al. Molecular markers of micrometastasis in oral cavity carcinomas. Otolaryngol Head Neck Surg 2009;141:52-8). It is shown that knock-down of CD44 greatly diminishes tumor growth in nude mice (Figure 1A) (P<0.05) (Perez A, et al. CD44 interacts with EGFR and promotes head and neck squamous cell carcinoma initiation and progression. Oral Oncol. 2013; 4:306-13). Figure 1B shows that Epidermal Growth Factor Receptor (EGFR), a major molecular driver of HNSCC, and its phosphorylated form (Y1068) are reduced on CD44-siRNA xenografts indicating that the two molecules are functionally related.

Because of the known role of CD44 in tumor initiation (Prince ME, et al. Identification of a subpopulation of cells with cancer stem cell properties in head and neck squamous cell carcinoma Proc Natl Acad Sci USA 2007;104:973-8), solCD44 was evaluated in oral rinses from cancer patients and controls. It has been shown that the test accurately distinguishes HNSCC from normal controls and those with benign disease of the head and neck (Franzmann EJ, et al. Salivary soluble CD44: a potential molecular marker for head and neck cancer. Cancer Epidemiol Biomarkers Prev 2005;14:735-739; Franzmann EJ, et al. Soluble CD44 is a potential marker for the early detection of head and neck cancer. Cancer Epidemiol Biomarkers Prev 2007;16:1348-1355; Franzmann EJ, et al. Salivary protein and solCD44 levels as a potential screening tool for early detection of head and neck squamous cell carcinoma. Head Neck. 2012; 34:687-95). It has also been discovered that total protein, measured by a simple Lowry-like assay, was elevated in HNSCC compared to controls (Franzmann EJ, et al. Salivary protein and solCD44 levels as a potential screening tool for early detection of head and neck squamous cell carcinoma. Head Neck. 2012; 34:687-95) and that combined solCD44 and total protein levels more effectively distinguish HNSCC from controls than either marker alone (Franzmann EJ, et al. Salivary protein and solCD44 levels as a potential screening tool for early detection of head and neck squamous cell carcinoma. Head Neck. 2012; 34:687-95; Pereira LH, et al. Salivary markers and risk factor data: a multivariate modeling approach for head and neck squamous cell carcinoma detection Cancer Biomark. 2011;10:241-9).

### Multi-Institutional, Blinded, Frequency-Matched, Case-Control Study (n=300):

150 oral cancer cases and 150 frequency matched, clinic-based controls were enrolled. Controls were enrolled between 2007 and 2012 from private primary care clinics on the University of Miami medical campus and from Jefferson Reaves, Sr. Health Center, an inner city county clinic operated by Jackson Memorial Hospital System. Study personnel were blinded to case-control status when running the solCD44 and protein assays. Eligibility decisions were made by the PI prior to unblinding.

The entire cohort of cases and controls (n=300) was at high-risk for tobacco-associated malignancy since 78% had a smoking history and approximately 50% admitted to moderate or heavy drinking. Approximately 40% from both groups had 6 or more teeth missing due to poor oral health. The case group included 53% JMH (county hospital) subjects, 44% were over 60 years old, 81% were male, 17% were black, and 51% were Hispanic. There were no significant differences (p<0.05) between cases and controls with respect to key covariates including age, gender, race, ethnicity, oral health (number of teeth removed), history of ever smoking, alcohol habit or numbers enrolled from the county versus private hospital system.

Both solCD44 and total protein levels were elevated in cases compared to controls (solCD44: 5.50ng/mL vs. 2.87ng/mL, p<0.0001; protein: 0.94mg/mL vs. 0.76 mg/mL, p=0.007). Marker levels were examined based on differences in risk factors or demographic variables between and within the case and control groups. SolCD44 and protein levels were generally higher in cases compared to controls at the p<0.05 level when age, gender, race/ethnicity, smoking habit or drinking habit were considered. A significant difference in solCD44 levels based on age (higher levels in older patients) were found in the case group.

| **Table 3. Logistic regression models for clinic based cohort** | **Odds Ratio (95%CI)** | ***P*** | AUC | **Rescaled R²** |
|---|---|---|---|---|
| **150 cases / 150 controls** | | | | |
| Log 2 CD44, 1 unit increase | 2.299 (1.605, 3.293) | <.0001 | 0.691 | 0.1430 |
| Protein, 1-unit increase | 0.738 (0.370, 1.475) | 0.3901 | | |
| Age, 1-yr increase | 0.988 (0.965, 1.013) | 0.3445 | | |

Tumor characteristics such as stage and p16 status (surrogate for HPV measured when tissue was available) (Lin, et al. Long-term prognosis and risk factors among patients with HPV-associated oropharyngeal squamous cell carcinoma. Cancer. 2013) did not significantly impact solCD44 or protein levels with the exception that a difference by tumor size (T1-T2 vs. T3-T4; CD44: 4.23 ng/mL vs. 6.4 ng/mL, p=0.026) was seen for CD44 but not for logCD44. Based on these findings, a logistic regression model was developed to distinguish HNSCC from controls based on log2 solCD44, protein and age. Despite a very high-risk and diverse cohort, the model had an accuracy of 0.69 as measured by Area Under the Curve (AUC) (Table 3).

African-Americans (males in particular) suffer disproportionately from oral cancer with lower overall survival rates. Subset analyses was performed by race, ethnicity and gender using multivariate logistic regression models including solCD44, protein and age to evaluate differences between populations. The results for the black male subset show that the model accurately distinguishes HNSCC patients from controls with AUC=0.89 (Table 4). Probability scores based on the regression model were calculated for each black male subject. An observation was predicted as case if the predicted probability exceeded or equaled 0.3418. At this cutpoint, sensitivity was 100% and specificity was 71.4% with accuracy at 87.1%.

| **Table 4. Logistic regression models for clinic-based black cohort** | **Odds Ratio (95% CI)** | **P** | **AUC** | **Rescaled R²** |
|---|---|---|---|---|
| **Black Males: 17 cases / 14 controls** | | | | |
| Log 2 CD44, 1 unit increase | 2.266 (0.702, 7.315) | 0.1711 | 0.889 | 0.5738 |
| Protein, 1-unit increase | 2.968 (0.321, 27.452) | 0.3377 | | |
| Age, 1-yr increase | 1.228 (1.053, 1.431) | 0.0087 | | |

For the other racial/ethnic and gender groups the prediction models including solCD44, protein and age resulted in the following AUCs: white non-Hispanic males=0.76; white Hispanic males=0.76; all females=0.64. The odds ratios for individual model components did not reach significance, likely due to small sample size, with the exceptions of age for black males, and age, log solCD44 and protein for white, Hispanic males (Table 5 below). Thus protein levels in Hispanic males, unlike black males, was associated with a significant protective effect.

| **Table 5. Logistic regression models for white Hispanic males** | **Odds Ratio (95%CI)** | ***P*** | **AUC** | **Rescaled R²** |
|---|---|---|---|---|
| **White Hispanic males: 59 cases / 75 controls** | | | | |
| Log 2 CD44, 1 unit increase | 5.098 (2.507, 10.368) | <.0001 | 0.756 | 0.2668 |
| Protein, 1-unit increase | 0.183 (0.059, 0.562) | 0.0030 | | |
| Age, 1-yr increase | 0.953 (0.914, 0.993) | 0.0231 | | |

***Community Cohort Early Detection and Prevention Study:*** A Bankhead-Coley (BHC) Research Project Grant was used to study the solCD44/protein test in Community Cohort. 150 black subjects were enrolled and tested, and baseline marker levels obtained. The BHC subjects were similar to black cases from the clinic-based trial with respect to risk factors and demographics except that they were a decade younger on average (51 years vs. 61 years, p<0.05) and there was a smaller proportion of Hispanic blacks in the BHC study (<1% vs. 27% p<0.05). The model and cutpoint derived for black males in the clinic-based study (Table 5) was applied to black males in the BHC study and it was found that 81 (92.05%) were predicted as controls and 7 (7.95%) as cases. One of these seven "false positives" developed lung cancer diagnosed by biopsy 14 months after baseline positive solCD44/protein test. Another subject had a history of prostate cancer and a normal baseline exam but high probability marker results. He developed a worrisome leukoplakia 9 months after oral rinse collection that progressed on the following annual visit. Marker levels in this subject are rising and biopsy is pending. Thus even some of the "false positives" were likely true positives with developing or distant disease.

The marker test is more specific in the community-based BHC cohort than the clinic-based cohort (92% vs. 71.4%). SolCD44 levels were lower in the BHC cohort than the black clinic-based control cohort, reaching borderline significance for log2 CD44 (CD44: 1.85 ng/mL vs. 2.67 ng/mL, log2CD44: 0.69 ng/mL vs. 1.14 ng/mL; p=0.057). This shows that the clinic-based cohort is a higher risk group than the community-based cohort. Supporting this, it was found that over 10% of the 150 clinic-based subject's verses less than 1% of the BHC cohort had a history of prior cancer. Clinic-based controls with a prior history of cancer had significantly increased solCD44 (median 3 vs. 2.2 ng/mL, p<0.05) and protein (median 1.03 vs. 0.64 mg/mL, p<=0.05) levels compared to controls without prior cancer.

There were 9 female black cases in the clinic-based study due to frequency matching criteria. A model with both genders also showed favorable AUC (Table 6).

| **Table 6. Lo gistic regression models for all black clinic-based cases vs. BHC controls** | **Odds Ratio (95%CI)** | ***P*** | **AUC** | **Rescaled R²** |
|---|---|---|---|---|
| **Black: 26 R01 cases vs. 150 BHC controls** | | | | |
| log₂ solCD44 | 4.741 (1.890, 11.894) | .0001 | 0.911 | 0.5630 |
| Protein in Males | 3.754 (0.950, 14.835) | .059 | | |
| Protein in Females | 0.195 (0.013, 3.024) | .242 | | |
| Age (Protein×Gender p= 0.0506, gender p=0.0502) | 1.235 (1.119, 1.363) | .0001 | | |

***SolCD44*/*protein test detects cancer before it is visible:*** Two control subjects from an earlier study (18) with elevated oral rinse solCD44 levels and no disease (false-positive), developed severe dysplasia and invasive cancer 2-3 years later. Two more clinic-based control participants who were at risk based on a regression tree model developed carcinoma in situ or invasive cancer in the follow-up period. A patient from the BHC study now has developed lung cancer and had elevated marker levels 14 months before diagnosis. Another subject with a worrisome oral lesion, history of prostate cancer and rising solCD44 and protein levels is pending oral biopsy.

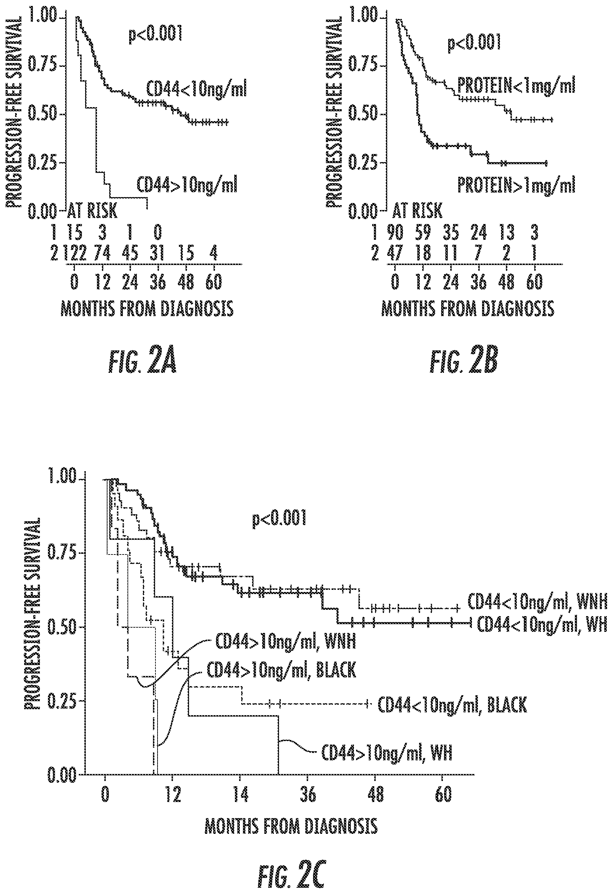

***Elevated solCD44 and protein levels are associated with poor prognosis:*** Figure 2 A-C shows PFS for 137 of 150 clinic-based cases for which there was a follow-up. There were 59 deaths out of 149 evaluable subjects (median follow-up 22.9 months, range: 0.7 to 65.1 months) and 68 progression events out of 137 evaluable subjects. Mean 3-year PFS and overall survival (OS) was 48.8% and 54.7% respectively. High levels of solCD44 (>10 ng/mL) and total protein (>1 mg/mL) were associated with decreased PFS. In univariate analysis, predictors of PFS and also of OS were CD44 (as continuous/categorical), protein (continuous/categorical), stage, T4 stage, race (black race worse outcome), and age. Neither gender, smoking and alcohol status, nor site (oropharynx v. oral cavity) were significant predictors. P16 was only available for a subset of cases; the effect of p16- vs. p16+ was significant for PFS (HR 2.201, p=0.0458, n=73) but not for OS (HR 1.531 p=0.2941, n=79). Multivariate analysis, excluding p16, using stepwise selection forcing CD44 and protein in models resulted in a common model for PFS and OS including: CD44, protein, stage, race, and age. Under this model, there was an independent risk effect of solCD44 level ≥10 on both PFS (HR 2.628, 95% CI: 1.325, 5.210, p=0.0057) and OS (HR 2.103, 95% CI: 1.031,4.291, p=0.0411). The risk effect for protein ≥1mg/mL approached significance for PFS (HR 1.571, 95% CI: 0.906, 2.725, p=0.1079) but was not significant for OS (HR 1.459, p=0.215). These findings show that elevated solCD44 and protein levels are associated with more aggressive disease. Thus the oral rinse test can identify disease that is most in need of early detection.

*Screening for HNSCC using the oral rinse test detects cancers at distant sites:*
SolCD44 and protein levels were elevated in clinic-based controls with prior history of cancer at other sites including prostate (7), colon (2), bladder (2), melanoma (2), leukemia/lymphoma (1), breast (1), and osteosarcoma (1) supporting that the markers can reflect risk of cancer outside the UADT. One of the BHC controls developed lung cancer 14 months after an elevated oral rinse marker test. In the clinic-based study, case and control participants were excluded from the main analysis if found to have a cancer in a site outside of the UADT at the time of collection. Two cases and 3 controls were excluded for this reason. One case with both colon and HNSCC had solCD44 levels of 64.2 ng/mL which is 22 times the normal level. One of 3 controls with distant cancers had bladder cancer with solCD44 levels of 14ng/mL and protein levels of 1.5 mg/mL. Another control, excluded due to possibility of low-grade oral premalignancy had solCD44 levels of 4.8 ng/mL and protein levels of 1.3 mg/mL and went on to develop lung cancer. This data shows that solCD44 and protein levels can indicate disease at more distant sites before it is recognized clinically.

### Experimental Procedure 1

*Community Cohort enrollment and screening sites:* Subjects were enrolled a food bank in Community Cohort that serves 1000 individuals weekly as well as a housing project. The Sylvester Cancer Center's Disparities and Community Outreach Core (DCO) facilitated the success of this community-based partnership.

*Subjects:* Enrollment of 150 subjects was completed in 2 years. The patients were enrolled if they had a history of smoking 100 cigarettes or more in their lifetime and if they were over 40 years old. The average age was 51.2 years, 58.7% were male, 100% were black and 99.3% were non-Hispanic. 117 participants were enrolled. Of those, 74 have returned for 1st and or 2nd annual follow-up and 5 are still in the window for follow-up.

*Questionnaire:* Following consent, the clinical research personnel administered a questionnaire to collect detailed information about potential covariates that may impact interpretation of results. The questionnaire is based on the BRFSS survey and includes queries on age, race, ethnicity, gender, tobacco and alcohol use, socioeconomic status (SES), education, nutrition and oral health (see Survey Instruments). All of these are implicated as possible risk factors for head and neck cancer. Patients are queried regarding symptoms related to and history of benign disease of the UADT and perform a review of organ system diseases including cancer. Participants list their prescription medications, herbal remedies, over-the-counter medication and vitamins and queried as to when they last used tobacco products or mouthwash, brushed their teeth or had anything to eat or drink.

*Head and neck exam:* Following administration of consent and the questionnaire, the PI, a trained head and neck surgeon, performs a standard head and neck exam including headlight-assisted inspection, palpation of the oral cavity, base of tongue and neck, cranial nerve exam, nasal exam and laryngeal mirror exam. White or red, raised or ulcerated lesions that may represent malignancy are recorded and referred for biopsy. Other abnormalities such as reflux changes and infections are noted with referral as indicated. Patients with reactive lesions and ulcerations that do not appear worrisome enough to warrant biopsy are followed at the Community Cohort site while those in need of treatment or biopsy are referred to the UM or JMH head and neck clinic. Relative amount of tooth decay and periodontal disease are considered and scored on a scale from 0 to 2. All abnormalities along with dates of diagnosis are documented for database input.

*Collection and processing of oral rinse:* Oral rinse can be used instead of serum measurements because solCD44 levels in serum are largely contaminated by variant isoforms derived from normal epithelial compartments (Van Hal NL, et al. Evaluation of soluble CD44v6 as a potential serum marker for head and neck squamous cell carcinomal Clin Cancer Res 1999;5:3534-41). The oral rinse is mainly composed of saline with a small amount of saliva and contacts the UADT mucosa. The research personnel performs collection of oral rinses from subjects at the screening site. Subjects are asked to refrain from oral hygiene procedures, smoking, eating and drinking for at least 1 hour prior to collection (Navazesh M. Methods for collecting saliva. Ann NY Acad Sci 1993;694:72-7). HNSCC patient's gargles were scored on a scale from 0 to 2. Following collection, samples are refrigerated, transferred on ice to the laboratory, centrifuged, the pellet is separated, rinse samples are fractioned and these fractions and the pellet are stored at -80°C.

*Follow-up:* Annual oral rinse collections, questionnaires and physical exams were performed through the duration of the study.

*Data collection:* Findings on exams are recorded using preprinted logs and entered into the database. Questionnaires, logs, and oral rinse samples contain no identifying information, but are labeled with a code unique for that subject. A master log links identifying information with this number. This master log is kept separate from databases used for analysis to maintain patient confidentiality and blinding while performing assays.

*SolCD44 and protein tests:* The solCD44 ELISA test was performed according to the instructions supplied by the manufacturer (Bender MedSystems) with modifications as described in multiple publications (17-20). Samples are tested in batches and measured at full concentration. The protein assay (Bio-Rad Laboratories) was performed according to the manufacturer's protocol as previously published (Franzmann, et al. Salivary soluble CD44: a potential molecular marker for head and neck cancer. Cancer Epidemiol Biomarkers Prev 2005;14:735-739; Franzmann, et al. Soluble CD44 is a potential marker for the early detection of head and neck cancer. Cancer Epidemiol Biomarkers Prev 2007;16:1348-1355; Franzmann, et al. Salivary protein and solCD44 levels as a potential screening tool for early detection of head and neck squamous cell carcinoma. Head Neck. 2012; 34:687-95; Pereira, et al. Salivary markers and risk factor data: a multivariate modeling approach for head and neck squamous cell carcinoma detection. Cancer Biomark. 2011;10:241-9). Results were entered by sample code into a database which is kept separate from the database containing patient information until analysis.

| **Table 7. Changes in CD44 over 1 year** | | | | | | | |
|---|---|---|---|---|---|---|---|
| **Year** | **N** | **Variable** | **MMean** | **Median** | **Minimum** | **Maximum** | **Std Dev** |
| 0 | 51 | CD44 | 1.634 | 1.335 | 0.505 | 4.595 | 0.886 |
| 1 | 51 | CD44 | 1.484 | 1.150 | 0.265 | 5.325 | 1.123 |
| | | Difference | -0.150* | -0.260 | -2.758 | 3.810 | 1.184 |
| * Significantly different from zero (p=0.0185) Mann-Whitney U test. | | | | | | | |

*Quality Control:* Samples that test outside the accepted ranges are repeated based on defined quality control criteria. For example, the absorbance of the highest standard may range from 1.5-2.5. Individual sample and intraplate variation is accepted up to 10% CV. Interplate variability up to 20% CV is allowed. Levels above the highest standard are repeated at ½ concentration. All repeated measures (e.g. each annual collection for an individual subject) are performed on the same day and ELISA plate to reduce technical variability.

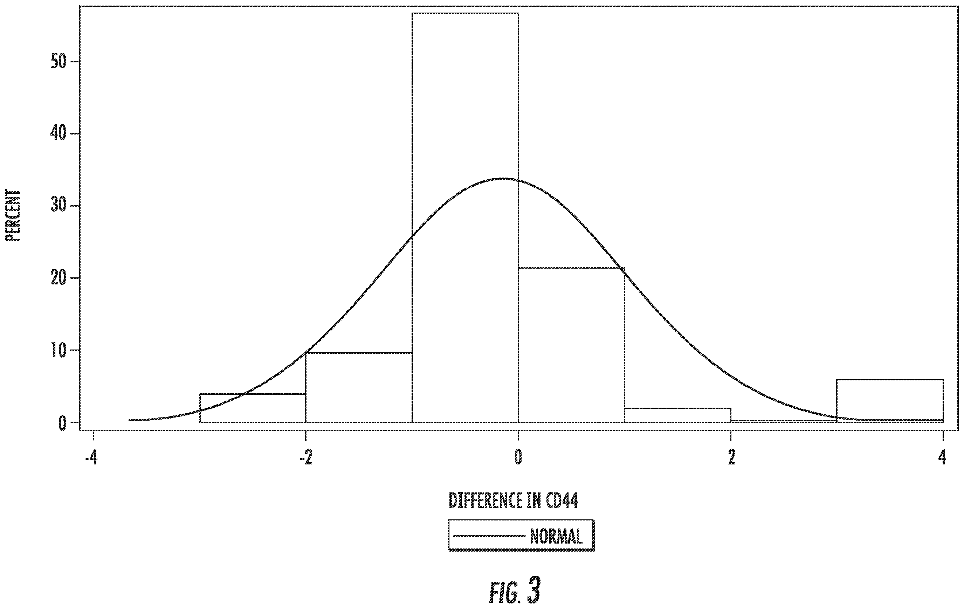

*Example Data:* Marker level results were obtained at baseline and first annual visit for 51 BHC subjects. The changes were quantified in individual participant's marker levels over the one year period and then plotted these changes forming a histogram. Overall, for solCD44, log2CD44 and protein, the highest percentage of participants tended to have fluctuations closer to the zero median. Annual mean solCD44 levels decreased from 1.63 to 1.48 ng/mL (p=0.0185) (Figure 3 and Table 7) showing that risk is decreasing. This change in CD44 was accompanied by a significant change in log2CD44 while protein did not change at all

*Statistical analysis:* This is a longitudinal study of 150 subjects followed annually over a total period of 5 years. The levels of solCD44 and protein can be collected at enrollment and up to 4 subsequent times. Unequal numbers of observations per subject with unequal spacing over time are expected, and the analysis methods are designed to account for this. Marker data is log-transformed as needed to stabilize variance and improve a normal distribution fit. Time-series plots are used to describe individual and overall trajectories *e.g.,* changes in CD44 expression are depicted by plotting the mean level of study subjects together with individual subject values as a function of time overall and by explanatory variables. General mixed effects models for repeated measures are used to describe changes in marker levels over time, with adjustment for explanatory variables such as gender, age etc. Significance of interactions between time and explanatory variables expected are that different patterns in marker expression over time can be found for subgroups defined by some of these variables. Analyses are conducted in SAS® 9.3 using The PROC MIXED procedure for random effects models. The structure of the variance-covariance matrix representing the dependence between measurements are chosen according to standard statistical criteria. Post-hoc pairwise comparisons between times, and between subgroups at selected times, are performed with adjustments to preserve the overall Type I error rate.

*Study power illustration*: For simplicity, power illustration is shown based on 150 subjects, two repeated observations per person, and two groups (*e.g.*, smoking categories). The overall study size of 150 subjects comparing paired data (*e.g.* baseline vs. 1 year follow-up) have 80% power is used to detect an effect size of 0.23, based on a paired t-test at two-sided significance level of 5%. For a comparison between two times within a smaller group size of 100 or 50 subjects (*e.g.* current smokers), a 80% power is used to detect an effect size of 0.28 or 0.40, respectively. For a two-sample comparison, *e.g.* 50 current smokers vs. 100 former smokers at a given time (*e.g.,* at enrollment), based on a two-sample t-test at two-sided significance level of 5%, there is 80% power to detect an effect size of 0.56.

| **Table 8 CD44 and Protein Levels in Confirmed Tobacco Quitters** | | | | |
|---|---|---|---|---|
| **IDs** | **Baseline CD44 (ng/mL)** | **Post Quit CD44 (ng/mL)** | **Baseline Protein (mg/mL)** | **Post Quit Protein (mg/mL)** |
| 13 | 1.20 | 1.32 | 0.21 | 0.20 |
| 16 | 3.61 | 1.45 | 0.60 | 0.30 |
| 28 | 0.51 | 1.16 | 0.16 | 0.41 |
| 58 | 1.48 | 1.15 | 0.49 | 0.42 |
| 59 | 1.20 | 2.03 | 0.15 | 0.62 |
| 65 | 1.00 | 1.42 | 0.36 | 1.05 |
| 69 | 0.94 | 2.09 | 0.06 | 0.30 |
| 83 | 2.50 | 2.65 | 1.29 | 0.81 |
| 88 | 0.90 | 0.65 | 0.30 | 0.20 |
| 117 | 0.45 | 1.55 | NA | NA |
| 118 | 2.04 | 5.45 | 0.24 | 0.69 |

### Experimental Procedure 2

An ideal early detection test identifies HNSCC at a reversible stage. This poses a challenge as early disease is often invisible (Poh CF, et al. Direct fluorescence visualization of clinically occult high-risk oral premalignant disease using a simple hand-held device Head Neck. 2007 Jan;29(1):71-6). Molecular imaging may help, but without a discrete lesion, there are no ablative options for the patient. Thus it is optimal to have an intervention that reduces risk in subjects where a lesion is not yet identified. There is evidence to support that CD44 isoforms in serum decrease following smoking cessation. In experimental procedure 2, it is shown that solCD44 and protein levels in oral rinses decrease with smoking cessation.

A total of 125 subjects were enrolled. Of those, 23 said that they quit using tobacco at the 3 month and 15 at the 1 year mark. Of quitters for at least 3 months cotinine confirmation has been obtained on 15. The available results of solCD44 and protein levels are given in Table 8. Most of these subjects have marker levels at or below average for controls (solCD44=1.85ng/mL, protein=0.63mg/mL).

*Subjects:* Subjects are those who successfully quit for at least 3 months but with no cancer or premalignancy. Participants are recruited primarily thorough community organizations in the Community Cohort area and through county-wide recruitment efforts. Smokers who are attempting to quit regardless of whether they are enrolled in intervention programs (e.g., group or individual counseling, nicotine replacement, etc.) are enrolled. Those over 40 who are at higher risk of HNSCC are preferentially enrolled.

*Smoking cessation program:* Smokers are referred to resources that meet their needs (*e.g.,* Florida Smokers' Quit line, primary care, self-help materials, or counseling programs). Current smokers who are interested in behavioral treatment combined with nicotine replacement are referred to the UM clinic for intervention. The program includes intensive, group-based counseling using cognitive behavioral strategies. Participants are followed as they complete their preferred cessation program and maintain contact with participants by telephone and mail.

Oral rinse, saliva sample, and questionnaire: Subjects are provided an oral rinse, saliva sample and complete a questionnaire at the smoking cessation clinic prior to quitting. Serum solCD44 levels drop by 20-30% within the first 4 weeks following smoking cessation, therefore subjects who have stopped smoking more than a week prior to collection are excluded (Scott DA, et al. Plasma concentrations of reputed tumor-associated soluble CD44 isoforms (v5 and v6) in smokers are dose-related and decline with smoking cessation. 2000 Cancer Epidemiol Biomark Prev 2000;9:1211-4). Whole, unstimulated saliva is collected into a specimen cup for 5 minutes. The saliva samples are refrigerated until analysis. Collections and questionnaires are administered at baseline (prior to quitting), and at 3 months and 1 year following quitting.

*SolCD44, protein and cotinine assays:* Cotinine is a major metabolite of nicotine and has been widely used as a biomarker of tobacco exposure. The salivary cotinine level is measured using a kit (Salimetrics) according to the manufacturer's instructions. SolCD44 and protein assays are performed accordingly

*Follow-up:* Patients are followed in the smoking cessation clinic. An oral rinse and questionnaire at the three time points.

*Data collection:* Data pertaining to smoking cessation including, dates of sample collection and questionnaire administration, quit start and quit end dates, are recorded on logs and entered into the database using privacy protection.

*Statistical Analysis:* The levels of solCD44, protein and cotinine are collected from all study subjects at enrollment into a smoking cessation program at 3 months and at 1 year following quitting. Quitters vs. nonquitters are compared with respect to marker level after completion of the smoking cessation program using two-sample t-test, or multiple regression models to allow adjustment for explanatory variables. Analysis of longitudinal data for the markers of interest in successful quitters is similar to that described above starting with subject and group plots of marker levels as a function of time.

*Study power illustration:* As an illustration of power, study size consisting of 30 quitters for a comparison of paired data (*e.g.* pre smoking cessation program vs. 3 months and 1 year after completion) have 80% power to detect an effect size of 0.53, based on a paired t-test and a correlation between paired observations of 0.5 at two-sided significance level of 5%. The mean (and standard deviation) of log2sol CD44 measurements was 1.6 (0.7) among current smokers; thus the effect size 0.53 corresponds to a mean difference of 0.4 (0.86), that is a 25% reduction in log2sol CD44 (from 1.6 to 1.2). For the comparison quitters vs. nonquitters with respect to marker level at 3 months after completion of the smoking cessation program, based on a two-sample t-test at two-sided significance level of 5%, a 80% power to detect an effect size of 0.62, assuming study size of 30 quitters and 90 nonquitters.

### Experimental Procedure 3

*Inclusion Criteria:* Subjects (n=30) are included if they have newly diagnosed, biopsy proven squamous cell carcinoma of the oral cavity or oropharynx. Treatment (*e.g.*, surgery, chemotherapy, radiation) are noted and addressed in the statistical analysis. Subjects are enrolled from the Head and Neck clinics at UM and JMH. Diagnosis are confirmed by surgical pathology report.

*Exclusion criteria:* Patients with prior cancer of any histology involving the UADT are excluded. Patients with a history of primary squamous cell carcinoma in the hypopharynx, nasopharynx, paranasal sinuses, esophagus, salivary glands or in or below the larynx are excluded. Pregnant or nursing women are excluded. Patients with any prior history of cancer at another site except squamous or basal cell carcinomas of the skin are excluded.

*Oral rinse collection, solCD44 and protein assays, data collection:* All collection (including questionnaires) and assay procedures are as described herein. Complete AJCC staging information, pathologic characteristics including p16 status (per institutional standard), and type and duration of treatment given *e.g.* chemotherapy, radiation therapy, surgery are to be documented.

*Measurements of tumors before and after treatment:* Measurements are determined using standard RECIST (Response Evaluation Criteria in Solid Tumors) (Eisenhauer EA, et al. New response evaluation criteria in solid tumours: revised RECIST guideline (version 1.1). Eur J Cancer. 2009;45:228-47). Tumors must be measurable by standard imaging. Imaging time points are per institutional protocol.

*Time points for oral rinse collection:* Oral rinses are collected at diagnosis (baseline), and three months after completing therapy if radiation is given (since radiation continues to work for about 3 months after the last dose is given) and one month after completing therapy if no radiation is given.

*Statistical Analysis:* It was determined that solCD44 marker levels decrease with response to treatment. Data for analysis includes 1) results from oral rinses for solCD44 and protein and 2) tumor measurements before and after treatment, 3) descriptions of tumor characteristics, treatment, and risk factors (*e.g.* smoking). Quantitative variables (such as solCD44 and total protein) are summarized in terms of mean and standard deviations. Data are transformed as needed to improve a normal fit. Categorical variables are summarized as counts and percentages. How all markers change from before to after treatment is examined. This corresponds to a longitudinal design, where the levels of solCD44 and protein are collected pre- and post-treatment. General mixed effects models are used for repeated measurements to describe changes in marker levels pre- vs. post-treatment, with adjustment for covariates with the small sample size. Analyses are conducted in SAS® 9.3 using the PROC MIXED procedure for random effects models. The structure of the variance-covariance matrix representing the dependence between measurements are chosen according to standard statistical criteria. Pairwise comparisons between times, and between subgroups (*e.g.*, race categories) at specific times, are performed with adjustments to preserve the overall Type I error rate.

*Study power and precision:* Cases can have a complete response (CR) to treatment depending on their smoking status (Browman GP, et al. Influence of cigarette smoking on the efficacy of radiation therapy in head and neck cancer. N Engl J Med. 1993;328:159-63). For evaluation of marker level before to after treatment with in a subgroup of 15 CR patients (50% CR rate) can have 80% power to detect a small to moderate effect size of 0.67, based on a paired t-test at two-sided significance level of 5%. Assuming 21 CR patients (CR rate of 70%), there is 80% power to detect effect size of 0.55.

### Example 2: HPV Positive Squamous Cell Carcinoma, CD44, and HPV Positive Cervical Infection

### Methods

150 patients with HNSCC and 150 controls were enrolled according to protocol between January 2007 and September 2013. They were frequency matched for age, ethnicity, tobacco and alcohol use. They were enrolled from otolaryngology clinics and cancer centers. Control subjects were approached if they responded "yes" to tobacco or alcohol use on the clinic intake questionnaire. Controls were excluded if they had a potentially malignant condition. Case subjects were all patients with biopsy proven Head and Neck Squamous Cell carcinoma of all stages and sites. Those with nasopharyngeal carcinoma were excluded. Subjects were also excluded if pregnant, or infected with HIV.

A subgroup of 31 females with oropharyngeal cancer were identified, and 21 were tested for an HPV surrogate marker, p16, using IHC. Their charts were reviewed to verify oropharyngeal HPV status. Additional chart review was completed for assessing cervical HPV status via PAP smear records, and primary care notes. A subgroup of 34 female controls were identified. Additional chart review was completed for these subjects to assess cervical HPV status via PAP smear records, and primary care notes. PAP smear tests were considered positive if they showed evidence of at least atypical squamous cells of undetermined significance (ASCUS).

### Oral Rinse Collection

5 mL of normal saline was gargled for 5 seconds and swished for 5 seconds, then deposited into a specimen cup. Saliva was placed on ice for transport and stored at 80 degrees.

### Protein Assay

Total protein concentrations were determined using BioRad Protein Assay (BioRad, Hercules, CA). Protein estimation was carried out in duplicate.

### Soluble CD44 Assay

solCD44 concentration in oral rinse specimens were determined by using enzyme-linked Immunosorbent assay (Bender MedSystems, Vienna, Austria). All were variant isoforms of normal CD44. Samples were vortexed, centrifuged and the supernatant was used for the study. All experiments were performed in duplicate. Statistical analysis was performed by Mann-Whitney-Wilcoxon (MWW). Non-parametric test of the null hypothesis was performed.

### Results

### Demographics

The control group was made up of 34 individuals. There was 3% White Non-Hispanics, 6% Black Hispanics, 44% Black Non-Hispanics, and 47% White Hispanics. In the Case Group of 31 individuals, 29% were White Non-Hispanic, 26% were Black Non-Hispanic, and 45% were White Hispanic.

### Oropharyngeal Squamous Cell Carcinoma and p16 Status

In a group of 21 individuals, 36% were positive for p16. Of these p16 positive individuals, 4 were Black Non-Hispanic and 4 were White Hispanic. The remaining 64 % were negative for p16.

### Cervical PAP Smear

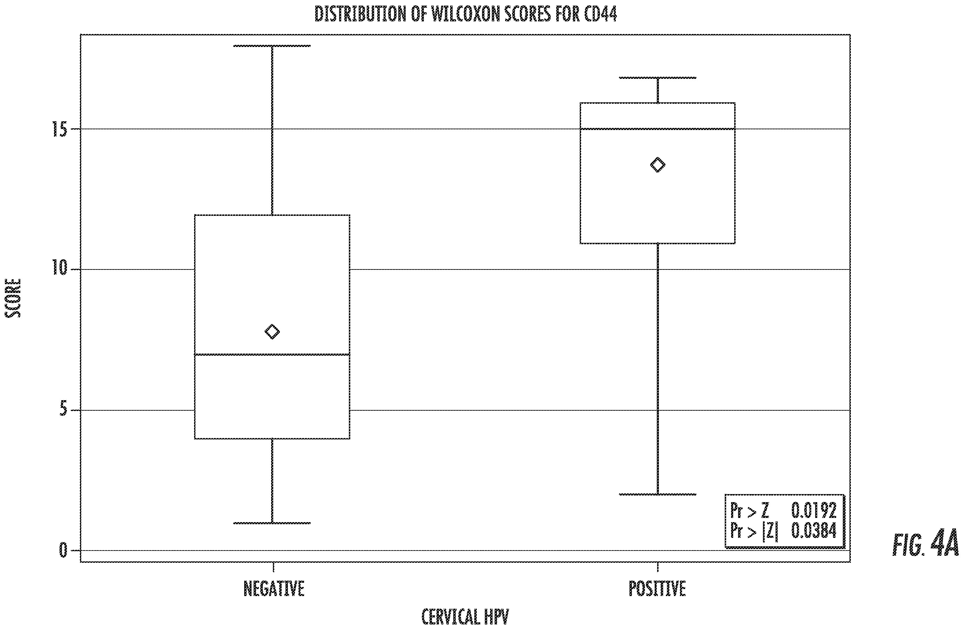

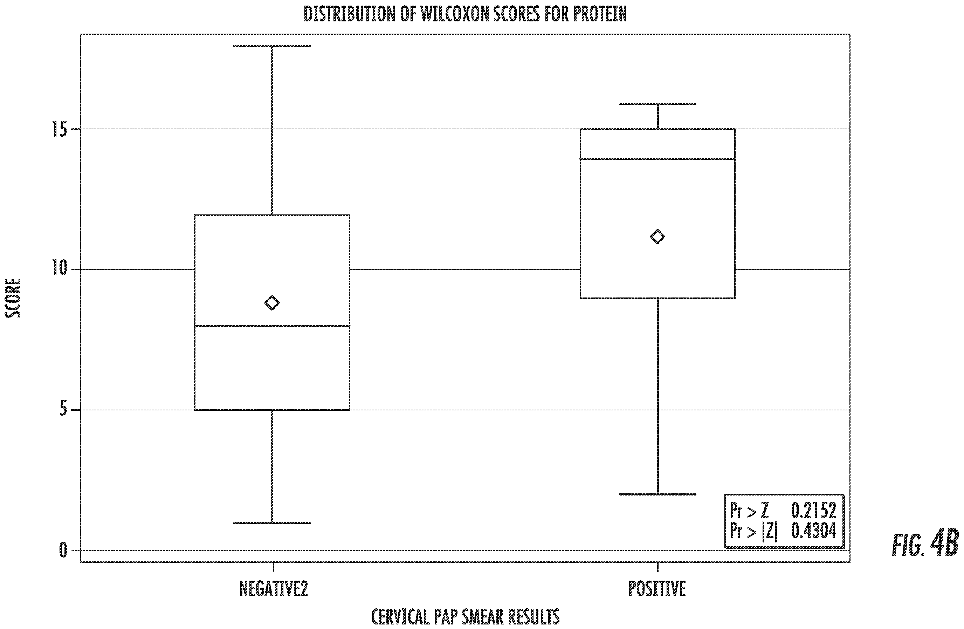

In the control group of 19 individuals, 26% had a positive PAP smear, while 74% had a negative PAP smear. In the case group of 8 individuals, 38% had a positive PAP smear, while 62% had a negative PAP smear. The differences in solCD44 and protein levels based on results of PAP smear in controls can be seen in Figures 4A and 4B.

### Discussion

Implement measure of p16 testing was performed on all patients with oropharyngeal squamous cell carcinoma. A Positive PAP Smear was shown as a potential risk factor for oropharyngeal squamous cell carcinoma. The incidence of positive PAP smears was higher in cases than controls. The levels of solCD44 are higher in patients with a positive PAP smear compared to those without a positive PAP smear.

Biron *et al.* demonstrated an increased risk of developing cervical cancer in oropharyngeal squamous cell carcinoma patients compared to general population. It was shown that the risk is at least 25 times greater, and presented more commonly with tonsillar tumors (55%) followed by tumor of the base of tongue (25%).Two previous studies examined the inverse association: the rate of second primary tumors in a large cohort of women with cervical cancer; and the risk of developing an oropharyngeal squamous cell carcinoma was increased compared with women in general population. The epidemiologic association reflects possible coinfection of HPV between cervix and oropharynx.

One case showed a patient with high risk HPV of the cervix and subsequent cervical cancer and p16 oral cavity cancer. Another patient with p16 oropharyngeal squamous cell carcinoma was p16 positive and HPV positive by in situ hybridization, with a history of hysterectomy 25 years prior.

### Example 3: Risk Stratification System for Oral Cancer Screening

A multi-institutional, case-control, hospital-based design was used to determine soluble CD44 (CD44) and total protein levels in oral rinses from 150 oral cancer patients and 150 controls frequency matched for age, gender, race, ethnicity, tobacco and alcohol use, and socioeconomic status (SES). Multivariate analysis was performed to determine associations between markers and case/control status. Progression-free (PFS) and overall survival (OS) were determined in cases. Subjects from an ongoing community-based oral cancer screening project (n=150), served as reference controls and levels were followed over 1 year to assess marker level variation.

Multivariate recursive partitioning stratified hospital-based subjects into 5 groups based on 2 cutpoints each for CD44 and protein. CD44 ≥5.33 ng/mL was highly associated with case status (adjusted OR 14.714, 95%CI: 6.094, 35.527; p<.0001, versus lowest risk group CD44 <2.22 ng/mL and protein <1.23 mg/mL as reference). Total protein aided prediction of case status when CD44 level was <5.33 ng/mL. CD44 ≥5.33 ng/mL was associated with poor PFS (adjusted HR=3.588, 95%CI: 1.558, 8.265, p=0.0027) and OS (adjusted HR=2.882, 95%CI: 1.165, 7.130, p=0.022). Marker levels dropped significantly (CD44:24%, p<.0001, protein: 16%, p=0.036) for subjects remaining in the community-based screening trial for 1 year (n=95).

### Study Protocol

Subjects were recruited from the University of Miami Sylvester Comprehensive Cancer Center (UM) and Jackson Memorial Hospital (JMH) Clinics between 2007 and 2012. All experiments were undertaken with the written consent of each subject according to The Code of Ethics of the World Medical Association (Declaration of Helsinki). This study evaluated soluble tumor markers in 150 oral cancer patients and 150 controls frequency matched for age, gender, race, ethnicity, tobacco and alcohol use, and socioeconomic status (SES). Oral cancer cases included newly diagnosed, previously untreated subjects with HNSCC involving the oropharynx (OP) and oral cavity (OC). Subjects were recruited equally from UM, a private university hospital system serving insured patients and JMH, a county hospital system serving primarily low-income patients. Subjects completed a baseline questionnaire, including demographics, behavioral risk factors and SES. For cases, data on tumor characteristics and outcomes were abstracted from medical records and the Tumor Registry. Controls with lesions worrisome for cancer were excluded. HIV positive or pregnant individuals were excluded. Exclusion decisions were made without knowledge of marker level results.

For validation we included a control cohort comprised of 150 individuals with history of tobacco or alcohol use from a low-income community in north Miami-Dade County. This community cohort was followed over time; baseline and annual follow-up oral rinses were obtained and measured to assess variation in marker in the screened population. Another control cohort of 21 normal volunteers were also included who were primarily nonsmokers. And last, 27 oral cavity and oropharyngeal cases and 39 controls with risk factors and history of benign diseases of the UADT whose levels had been tested as part of a previous hospital-based study were included (Pereira, et al. Cancer Biomark 2011;10:241-9).

### Laboratory Analysis

Oral rinse samples were collected using previously published procedures (Franzmann et al. Head Neck 2012;34:687-95; Pereira et al. Cancer Biomark 2011;10:241-9; Franzmann et al. Cancer Epidemiol Biomarkers Prev 2007;16:1348-55). Levels of solCD44 (normal and variant isoforms) were measured using a sandwich ELISA assay (eBioscience), with previously published modifications. The DC protein assay (Bio-Rad Laboratories) were performed according to the manufacturer's protocol using saliva samples prepared as previously published (Franzmann et al. Head & neck 2012;34:687-95; Pereira LH et al. Cancer Biomark 2011;10:241-9; Franzmann et al. Cancer Epidemiol Biomarkers Prev 2007;16:1348-55). Each sample was tested in duplicate and the technician was blinded to disease status. Absorbances were read in a microplate reader (Bio-Rad Laboratories) and concentrations were determined using a standard curve.

Formalin-fixed and paraffin-embedded specimens were retrieved from cases, where available (n=79). HPV status was assessed using p16INK4A immunohistochemistry (IHC) which is an accepted surrogate marker for HPV status (Hafkamp et al. International Journal of Cancer 2008;122:2656-64; El-Naggar et al. Head Neck. 2012, 34:459-61). p16INK4A was performed according to the manufacturer's IHC protocol on 68 specimens. HPV status was also assessed through chart review by means of IHC (n=10) or in situ hybridization (ISH) (n=1). All specimens were reviewed by a study pathologist (CG) who was blinded to the clinical data for the patients. p16INK4A expression was scored as positive if strong and diffuse nuclear and cytoplasm staining was present in ≥ 50% of the tumor specimen (El-Naggar et al. Head Neck. 2012;34:459-61).

### Statistical Analysis

Patient groups were compared with respect to the distribution of potentially important categorical covariates using the chi-square test or Fisher's exact test. Data on CD44 were log base-2 transformed to stabilize estimates of variance and improve the fit to the normal distribution. Continuous variables were analyzed using Student t-test or analysis of variance (ANOVA) followed by Fisher's least-significant-difference test for pairwise mean comparison, and tests of pre-specified contrasts. Logistic regression analysis was used to assess the association between markers and the risk for oral cancer. Odds ratio (OR) estimates were reported with corresponding 95% confidence interval (95%CI) and area under the curve (AUC) of the operating characteristic curve (ROC) for fitted models. Also, estimates of sensitivity, specificity, and accuracy derived from a fitted, multivariate logistic model which included significant interactions between markers and covariates as well as a model including risk groups based on cutpoints for CD44 and protein that were derived using multivariate recursive partitioning analysis (Breiman, et al. 1984 Classification and Regression Trees. Wadsworth, Belmont, CA) implemented in the R-packages MVPART (v.1.6.1.) and Recursive Partitioning and Regression Trees (RPART), version 1.6-0. Kaplan-Meier and Cox regression models were used to evaluate PFS and overall OS. Hazard ratio (HR) estimates and corresponding 95%CI were reported. Statistical analyses were performed using SAS version 9.2 (SAS Institute, Inc.) and R package.

### Results

### Characteristics of Hospital-Based Case-Control Study

The description of the hospital-based case-control study, comprising 150 patients with oral cancer and 150 controls, is summarized in Table 9.

**Table 9. Characteristics of cases and controls**

| Variable / Category | **Cases (n=150)** | | **Controls (n=150)** | | ***P*** |
|---|---|---|---|---|---|
| | N | % | N | % | |
| Site of enrollment | | | | | |
| **JMH** | 80 | 53.3 | 71 | 47.3 | 0.299 |
| **UM** | 70 | 46.7 | 79 | 52.7 | |

| Age, y | | | | | |
|---|---|---|---|---|---|
| **<40** | 4 | 2.7 | - | - | 0.214 |
| **40 \|- <50** | 20 | 13.3 | 29 | 19.3 | |
| **50 \|- <60** | 60 | 40.0 | 56 | 37.3 | |
| **60 \|- <70** | 44 | 29.3 | 44 | 29.3 | |
| **≥70** | 22 | 14.4 | 21 | 14.0 | |
| | | | | | |
| **<60** | 84 | 66.0 | 85 | 56.7 | 0.449 |
| **>=60** | 86 | 44.0 | 65 | 43.3 | |
| | | | | | |
| **Mean (SD)** | 58.6 (10.5) | | 58.5 (9.7) | | 0.887 |
| **Median (range)** | 58 (28 - 88) | | 58.5 (40 - 87) | | |

| Gender | | | | | |
|---|---|---|---|---|---|
| **Male** | 121 | 80.7 | 118 | 78.7 | 0.907 |
| **Female** | 29 | 19.3 | 32 | 21.3 | |

| Race | | | | | |
|---|---|---|---|---|---|
| **White** | 123 | 82.6 | 118 | 79.7 | 0.534 |
| **Black** | 26 | 17.4 | 30 | 20.3 | |
| **Asian/Other/Missing (1 case Other, 1 control Asian, and 1 control missing)** | 1 | | 2 | | |
| | | | | | |

| Ethnicity | | | | | |
|---|---|---|---|---|---|
| **Hispanic** | 77 | 51.3 | 93 | 62.0 | 0.062 |
| **Non-Hispanic** | 73 | 48.7 | 57 | 38.0 | |

| Oral health score | | | | | |
|---|---|---|---|---|---|
| **Poor/Fair** | 80 | 64.0 | 87 | 58.0 | 0.310 |
| **Good** | 45 | 36.0 | 63 | 42.0 | |
| **Missing** | 25 | | | | |

| Teeth removed | | | | | |
|---|---|---|---|---|---|
| **None/1 to 5** | 86 | 58.9 | 92 | 63.0 | 0.301 |
| **6 or more but not all** | 36 | 24.7 | 39 | 26.7 | |
| **All** | 24 | 16.4 | 15 | 10.3 | |
| **Missing** | 4 | | 4 | | |

| Smoking status | | | | | |
|---|---|---|---|---|---|
| **Ever** | 117 | 78.0 | 118 | 78.7 | 0.889 |
| **Never** | 33 | 22.0 | 32 | 21.3 | |

| Drinking habits¹ | | | | | |
|---|---|---|---|---|---|
| **Non-drinker/Mild** | 76 | 50.7 | 81 | 54.0 | 0.196 |
| **Moderate** | 26 | 17.3 | 34 | 22.7 | |
| **Heavy** | 48 | 32.0 | 35 | 23.3 | |

| | | | | | |
|---|---|---|---|---|---|
| ¹ Drinking habits: Non-drinker/Mild**: past drinking ≤2 drinks/day or current drinking ≤2 drinks/day for 1-15 days/month;** Moderate**: past drinking 3 to <5 drinks/day or current drinking ≤2 drinks/day for 16-30 days/month or ≥3 drinks/day for 1-15 days/month; Heavy: past drinking 5 or more drinks/day or current drinking ≥3 drinks /day for 16-30 days/month.** | | | | | |

There were no significant differences between cases and controls with respect to age, gender, race, oral health (number of teeth removed), history of ever smoking, alcohol habit or whether they were enrolled from the county (JMH) versus private hospital (UM) system. Table 10 shows cancer-specific characteristics among the cases. OP patients were more likely to present in late stage (III/IV vs. I/II; p<.0001), show more advanced N-status (N1-N3 vs. N0, Nx; p<.0001) and have HPV + vs. HPV - tumors (p<.001) compared to OC patients.

**Table 10. Stage and HPV status by disease site among 150 cases**

| **Variable/Category** | **All cases (N=150)** | | **OC cancer (N=59)** | | **OP cancer (N=91)** | | |
|---|---|---|---|---|---|---|---|
| | N | % | N | % | N | % | *P* |
| Stage | | | | | | | |
| Stage I/II | 26 | 17.3 | 20 | 33.9 | 6 | 6.6 | <.0001 |
| Stage III/IV | 124 | 82.7 | 39 | 66.1 | 85 | 93.4 | |

| T-stage | | | | | | | |
|---|---|---|---|---|---|---|---|
| T1-T2 | 63 | 42.0 | 28 | 47.5 | 35 | 38.5 | 0.276 |
| T3-T4 | 87 | 58.0 | 31 | 52.5 | 56 | 61.5 | |

| N-stage | | | | | | | |
|---|---|---|---|---|---|---|---|
| N0, Nx | 51 | 34.0 | 34 | 57.6 | 17 | 18.7 | <.0001 |
| N1, N2,N3 | 99 | 66.0 | 25 | 42.4 | 74 | 81.3 | |

| M-stage | | | | | | | |
|---|---|---|---|---|---|---|---|
| Mx | 24 | 16.0 | 7 | 11.9 | 17 | 18.7 | 0.316 |
| M0 | 123 | 82.0 | 50 | 84.7 | 73 | 80.2 | |
| M1 | 3 | 2.0 | 2 | 3.4 | 1 | 1.1 | |
| HPV by P16 | 79 | 100.0 | 28 | 100.0 | 51 | 100.0 | |

| IHC/ISH | | | | | | | |
|---|---|---|---|---|---|---|---|
| Positive | 31 | 39.2 | 4 | 14.3 | 27 | 52.9 | <.001 |
| Negative | 48 | 60.8 | 24 | 85.7 | 24 | 47.1 | |
| Missing | 71 | | 31 | | 40 | | |

Log₂CD44, hereafter referred to as CD44, and total protein were evaluated with respect to risk factors or demographic variables within the case and control groups (Table 11). CD44 and protein levels were higher in cases compared to controls at the p<0.05 level when age, gender, race/ethnicity, smoking habit or drinking habit, teeth loss or ability to gargle were considered. CD44 levels varied significantly with age (higher levels in older patients), gargle (higher with worse gargle ability), and teeth loss (higher with more teeth loss) in the case group but not in the control group. Levels of CD44 and protein did not differ significantly by TNM status or HPV status.

**Table 11 log₂solCD44, and protein levels in oral rinses of R01 HNSCC study by patient group and key variables**

| | | | log₂ solCD44 (mg/mL) | | | | P | Protein (mg/mL) | | | | P |
|---|---|---|---|---|---|---|---|---|---|---|---|---|
| | Cases | Controls | Cases | | Controls | | | Cases | | Controls | | |
| | N | N | Mean | SE | Mean | SE | | Mean | SE | Mean | SE | |
| All | 150 | 150 | 1.94^{a} | 0.09 | 1.28^{a} | 0.07 | <.0001 | 0.94^{a} | 0.05 | 0.76^{a} | 0.03 | 0.003 |

| Site of enrollment | | | | | | | | | | | | |
|---|---|---|---|---|---|---|---|---|---|---|---|---|
| JMH | 80 | 71 | 1.96^{a} | 0.14 | 1.32^{a} | 0.11 | <.0001 | 0.95^{w} | 0.07 | 0.81^{w} | 0.05 | 0.017 |
| UM | 70 | 79 | 1.92^{b} | 0.11 | 1.26^{b} | 0.09 | | 0.93^{a} | 0.06 | 0.73^{a} | 0.04 | |

| Age | | | | | | | | | | | | |
|---|---|---|---|---|---|---|---|---|---|---|---|---|
| <60 | 84 | 85 | 1.71^{a,c} | 0.12 | 1.16^{a,w} | 0.08 | <.0001 | 0.88^{w} | 0.06 | 0.75^{w} | 0.04 | 0.010 |
| 60 or more Gender | 66 | 65 | 2.24^{b,c} | 0.14 | 1.45^{b,w} | 0.12 | | 1.00^{a} | 0.07 | 0.78^{a} | 0.05 | |
| Male | 121 | 118 | 2.01^{a} | 0.10 | 1.29^{a} | 0.08 | <.0001 | 0.96^{a} | 0.05 | 0.80^{a} | 0.04 | 0.006 |
| Female | 29 | 32 | 1.68 | 0.21 | 1.28 | 0.16 | | 0.86^{w} | 0.10 | 0.64^{w} | 0.07 | |

| Race/Ethnicity (n=147) | | | | | | | | | | | | |
|---|---|---|---|---|---|---|---|---|---|---|---|---|
| White Non-Hispanic | 53 | 29 | 1.93^{a} | 0.15 | 1.31^{a} | 0.14 | <.001 | 0.91^{a} | 0.08 | 0.68^{a} | 0.07 | 0.017 |
| White Hispanic | 70 | 89 | 1.91^{b} | 0.13 | 1.32^{b} | 0.08 | | 0.91 | 0.07 | 0.81 | 0.04 | |
| Black | 26 | 30 | 2.06^{c} | 0.26 | 1.14^{c} | 0.19 | | 1.08^{b} | 0.12 | 0.71^{b} | 0.07 | |
| Smoking status Never | 33 | 32 | 1.72^{a} | 0.20 | 1.23^{a} | 0.13 | <.0001 | 0.96 | 0.14 | 0.76 | 0.06 | 0.027 |
| Ever | 117 | 118 | 2.01^{b} | 0.10 | 1.30^{b} | 0.08 | | 0.93^{a} | 0.05 | 0.76^{a} | 0.04 | |
| | | | | | | | | | | | | |
| Never | 33 | 32 | 1.72^{a} | 0.20 | 1.23^{a} | 0.13 | <.0001 | 0.94 | 0.14 | 0.76 | 0.06 | 0.080 |
| Former | 37 | 59 | 2.13^{b} | 0.18 | 1.31^{b} | 0.10 | | 0.98^{a} | 0.09 | 0.78^{a} | 0.06 | |
| Current | 80 | 59 | 1.95^{c} | 0.13 | 1.29^{c} | 0.12 | | 0.91^{w} | 0.05 | 0.75^{w} | 0.05 | |

| In current smokers: | | | | | | | | | | | | |
|---|---|---|---|---|---|---|---|---|---|---|---|---|
| <20 pack-years | 33 | 29 | 1.86^{a} | 0.18 | 1.07^{a} | 0.20 | 0.003 | 0.96 | 0.08 | 0.71 | 0.07 | 0.203 |
| ≥ 20 pack-years | 42 | 23 | 1.99^{w} | 0.19 | 1.51^{w} | 0.14 | | 0.84 | 0.08 | 0.81 | 0.09 | |
| Alcohol past Non-drinker | 35 | 40 | 2.08^{a} | 0.19 | 1.38^{a} | 0.13 | <.0001 | 1.00^{a} | 0.09 | 0.73^{a} | 0.05 | 0.018 |
| Drinker (Mild/Mod/Heav y) | 115 | 110 | 1.90^{b} | 0.11 | 1.25^{b} | 0.08 | | 0.92^{b} | 0.05 | 0.78^{b} | 0.04 | |

| Alcohol current (n=146) | | | | | | | | | | | | |
|---|---|---|---|---|---|---|---|---|---|---|---|---|
| Non-drinker | 84 | 72 | 1.87^{a} | 0.12 | 1.40^{a} | 0.10 | <.0001 | 0.97^{w} | 0.07 | 0.82^{w} | 0.05 | 0.010 |
| Drinker (Mild/Mod/Heavy) | 64 | 76 | 2.04^{b} | 0.15 | 1.17^{b} | 0.10 | | 0.91^{a} | 0.06 | 0.72^{a} | 0.04 | |

| Alcohol status | | | | | | | | | | | | |
|---|---|---|---|---|---|---|---|---|---|---|---|---|
| Never | 33 | 30 | 2.08^{a} | 0.20 | 1.39^{a} | 0.16 | <.0001 | 1.01^{a} | 0.10 | 0.75^{a} | 0.07 | 0.019 |
| Ever | 117 | 120 | 1.90^{b} | 0.10 | 1.26^{b} | 0.08 | | 0.92^{b} | 0.05 | 0.77^{b} | 0.04 | |

| Teeth removed | | | | | | | | | | | | |
|---|---|---|---|---|---|---|---|---|---|---|---|---|
| None/1 to 5 | 86 | 92 | 1.82^{a,d} | 0.11 | 1.26^{a} | 0.08 | <.0001 | 0.90^{a} | 0.05 | 0.74^{a} | 0.04 | 0.020 |
| ≥6, but not all | 36 | 39 | 1.79^{b} | 0.15 | 1.25^{b} | 0.14 | | 0.81^{b} | 0.06 | 0.76 | 0.07 | |
| All | 24 | 15 | 2.33^{c.d} | 0.26 | 1.43^{c} | 0.20 | | 1.05^{b} | 0.13 | 0.84 | 0.10 | |

| Gargle | | | | | | | | | | | | |
|---|---|---|---|---|---|---|---|---|---|---|---|---|
| Poor/ Fair | 38 | 12 | 2.23^{a.c} | 0.22 | 0.88^{a} | 0.36 | <.0001 | 1.13^{a, b} | 0.11 | 0.66^{a} | 0.13 | <0.00 1 |
| Good | 100 | 131 | 1.82^{b,c} | 0.10 | 1.29^{b} | 0.07 | | 0.85^{b} | 0.05 | 0.77 | 0.03 | |

| Cancer site | | | | | | | | | | | | |
|---|---|---|---|---|---|---|---|---|---|---|---|---|
| Lip/OC | 59 | | 2.12 | 0.15 | | | 0.132 | 0.98 | 0.09 | | | 0.490 |
| Oropharyngeal | 91 | | 1.83 | 0.11 | | | | 0.91 | 0.05 | | | |

| Stage | | | | | | | | | | | | |
|---|---|---|---|---|---|---|---|---|---|---|---|---|
| Stage I/II | 26 | | 1.78 | 0.17 | | | 0.425 | 0.90 | 0.09 | | | 0.719 |
| Stage III/IV | 124 | | 1.98 | 0.11 | | | | 0.94 | 0.05 | | | |
| T-stage | | | | | | | | | | | | |
| T1-T2 | 63 | | 1.76^{w} | 0.12 | | | 0.088 | 0.89 | 0.05 | | | 0.431 |
| T3-T4 | 87 | | 2.07^{w} | 0.13 | | | | 0.97 | 0.07 | | | |

| N-stage | | | | | | | | | | | | |
|---|---|---|---|---|---|---|---|---|---|---|---|---|
| N0, Nx | 51 | | 1.97 | 0.14 | | | 0.848 | 0.95 | 0.08 | | | 0.850 |
| N1-N3 | 99 | | 1.93 | 0.12 | | | | 0.93 | 0.06 | | | |

| HPV (n=81) | | | | | | | | | | | | |
|---|---|---|---|---|---|---|---|---|---|---|---|---|
| HPV+ | 31 | | 1.90 | 0.23 | | | 0.877 | 0.88 | 0.09 | | | 0.966 |
| HPV- | 50 | | 1.94 | 0.16 | | | | 0.89 | 0.07 | | | |
| P: p value from ANOVA global test of equality of all means. Same letter identify pairwise mean comparison within group or within a category of a key variable that was significant at the 5% level (letters a, b, c) or at the 10% level (letters w, y) by Fisher's least-significant-difference test. | | | | | | | | | | | | |

### Risk Modeling

In univariate analysis, CD44 and total protein distinguished cancer cases from controls with an odds ratio for 1-unit increase in CD44 of 2.036 (95%CI:1.552, 2.671, p<0.0001, AUC=0.68) and for 1-unit increase in protein of 2.159 (95%CI:1.288, 3.617, p<0.0035, AUC=0.59). The AUC was improved to 0.757 following adjustments for important variables and their interactions; The OR for CD44 increased to 2.668 (95%CI: 1.794, 3.968 p<.0001), while the odds ratio for protein become less than 1 and non-significant (OR=0.661, 95%CI: 0.312, 1.399, p=0.279) (Table 12 Panel A).

HPV+ tumors which are frequent in nonsmokers with oropharyngeal HNSCC, have a better prognosis compared to smoking and alcohol induced tumors. Findings for the analysis stratified by p16INK4A (surrogate for HPV status) were similar to the combined analysis (Hafkamp et al. International Journal of Cancer 2008;122:2656-64; El-Naggar et al. Head Neck. 2012, 34:459-61). In the HPV- group, protein levels were associated with a significant protective effect following multivariate analysis (Table 12 Panel B).

Multivariate recursive partitioning and logistic regression analyses were employed to better understand the relationship between CD44, protein and prediction of disease presence (Table 12 Panel C). The classification tree resulted in 5 significant risk groups based on 2 cut points each for CD44 and protein, classifying as "control" subjects in groups 1 (CD44 <2.22 ng/mL and protein <1.23 mg/mL, n=102), and 4 (CD44 ≥2.22 & <5.33 ng/mL and protein ≥0.558 mg/mL, n=116), and as "case" subjects in groups 2 (CD44 <2.22 ng/mL and protein ≥1.23 mg/mL, n=5), 3 (CD44 ≥2.22 & <5.33 ng/mL and protein <0.558 mg/mL, n=20), and 5 (CD44 ≥5.33 ng/mL, regardless of protein level, n=57) (Table 12 Panel C). CD44 ≥5.33 ng/mL, regardless of protein level, was highly associated with case status (OR=11.830, 95%CI: 5.279,26.508;p<0.0001, comparing group 5 versus 1 as the reference). Protein aided in determining magnitude of risk when CD44 was below 5.33 ng/mL. For instance, patients with CD44 <2.22 ng/mL and protein > ≥1.23 mg/mL (group 2)had a signficant higher risk of being a case (OR=10.069, 95%CI: 1.079, 93.93, p<0.05) compared to patients in group 1. Patients with CD44 ≥2.22 & <5.33 ng/mL and protein below 0.558 mg/mL (group 3) also had a significantly higher risk of being a case (OR=10.069, 95%CI: 3.103, 32.672, p=0.0001) as compared to group 1. For patients with CD44 ≥2.22 ng/mL & <5.33 ng/mL and protein ≥0.558 mg/mL (group 4), a lower but significant risk of being a case (OR=2.192, 95%CI: 1.247, 3.854, p=0.006) was observed. OR derived from a multivariate model including demographic and risk factors showed that CD44 level ≥5.33 ng/mL continued to be highly associated with case status (adjusted OR=14.714, 95%CI: 6.094, 35.527; p<.0001) and total protein level aided prediction of case status (Table 12 Panel C).

**Table 12. Prediction Models**

| **Standard logistic Regression** | **Odds Ratio (95%CI)** | ***P*** | **AUC** | **Resealed R²** | | | |
|---|---|---|---|---|---|---|---|
| Univariate (150 cases / 150 controls) | | | | | | | |
| log₂ solCD44 | 2.036 (1.552, 2.671) | <.0001 | 0.681 | 0.1366 | | | |
| Protein | 2.159 (1.288, 3.617) | 0.0035 | 0.590 | 0.0417 | | | |

| Multivariate model (149 cases/148 controls) | | | | | | | |
|---|---|---|---|---|---|---|---|
| log₂ solCD44 | 2.668 (1.794, 3.968) | <.0001 | 0.757 | 0.2608 | | | |
| Protein | 0.661 (0.312, 1.399) | 0.279 | | | | | |
| Adjusted for age (p=0.006), gender (p=0.0263), race/ethnicity (p=0.0023), age×race/ethnicity (p=0.003), alcohol (p=0.20), gender×alcohol (p=0.0165), and smoking (p=0.764) | | | | | | | |

| **Standard Logistic Regression, Stratified by HPV status** | | | | | | | |
|---|---|---|---|---|---|---|---|
| HPV negative (48 cases / 150 controls) | | | | | | | |
| Univariate | | | | | | | |
| log₂ solCD44 | 2.311 (1.561, 3.422) | <.0001 | 0.689 | 0.1463 | | | |
| Protein | 1.838 (0.888, 3.807) | 0.1012 | 0.562 | 0.0199 | | | |

| Multivariate model (148 controls)*: | | | | | | | |
|---|---|---|---|---|---|---|---|
| log₂ solCD44 | 3.911 (2.103, 7.274) | <.0001 | 0.753 | 0.2479 | | | |
| Protein | 0.222 (0.068, 0.724) | 0.0125 | | | | | |
| *Adjusted for age (p=0.0149), gender (p=0.0054), gender×age (p=0.0049), race/ethnicity (p=0.650), smoking (p=0.345) and alcohol (p=0.298) | | | | | | | |

| HPV positive (31 cases / 150 controls) | | | | | | | |
|---|---|---|---|---|---|---|---|
| Univariate | | | | | | | |
| log₂ solCD44 | 2.001 (1.291, 3.102) | 0.0019 | 0.667 | 0.0964 | | | |
| Protein | 1.882 (0.789, 4.492) | 0.1542 | 0.567 | 0.0180 | | | |

| Multivariate model (148 controls): | | | | | | | |
|---|---|---|---|---|---|---|---|
| log₂ solCD44 | 3.200 (1.550, 6.603) | 0.0017 | 0.761 | 0.1911 | | | |
| Protein | 0.427 (0.091, 2.001) | 0.2805 | | | | | |
| AUC: area under the ROC curve. Rescaled R²: coefficient of determination measured the dispersion explained by model. Odds ratios: 1-unit increase for continuous variables log2 CD44, protein, and age; race/ethnicity (WNH and Black vs. WH), gender (Male v. Female), smoking and alcohol (Ever v. Never), and teeth removed (6 or more or all vs. 5 or less). | | | | | | | |

| **Multivariate Recursive Partitioning & logistic regression** | | | | | | | |
|---|---|---|---|---|---|---|---|
| **Univariate analysis of Risk Groups** | | | | | | | |
| Risk group | SolCD44 | Protein | Odds Ratio (95%CI) | Prediction | P | AUC | Rescaled R² |
| 1 (n=102) | < 2.22 | < 1.23 | Reference | control | | 0.722 | 0.2266 |
| 2 (n=5) | < 2.22 | ≥ 1.23 | 10.069 (1.079, 93.93) | case | 0.0427 | | |
| 3 (n= 20) | ≥ 2.22 & < 5.33 | < 0.558 | 10.069 (3.103, 32.672) | case | 0.0001 | | |
| 4 (n=116) | ≥ 2.22 & < 5.33 | ≥ 0.558 | 2.192 (1.247, 3.854) | control | 0.0064 | | |
| 5 (n=57) | ≥ 5.33 | | 11.830 (5.279, 26.508) | case | <.0001 | | |

| **Multivariate Analysis of Risk Groups** | | | | | | | |
|---|---|---|---|---|---|---|---|
| Risk group | SolCD44 | Protein | Odds Ratio (95%CI) | Prediction | P | AUC | Rescaled R² |
| 1 (n=102) | < 2.22 | <1.23 | Reference | control | | 0.783 | 0.3150 |
| 2 (n=5) | < 2.22 | ≥ 1.23 | 5.990 (0.597, 60.112) | case | 0.1282 | | |
| 3 (n= 20) | ≥ 2.22 & < 5.33 | < 0.558 | 12.930 (3.627, 46.094) | case | <.0001 | | |
| 4 (n=116) | ≥ 2.22 & < 5.33 | ≥ 0.558 | 2.728 (1.475, 5.047) | control | 0.0014 | | |
| 5 (n=57) | ≥ 5.33 | | 14.714 (6.094, 35.527) | case | <.0001 | | |

| **Covariates for Multivariate Analysis** | | | | | | | |
|---|---|---|---|---|---|---|---|
| White Non-Hisp vs Black at age <60 | | | 6.307 (1.974, 20.153) | | | | |
| White Hisp vs Black at age <60 | | | 1.744 (0.631, 4.824) | | | | |
| White Non-Hisp vs Black at age ≥60 | | | 0.518 (0.154, 1.743) | | | | |
| White Hisp vs Black at age ≥60 | | | 0.338 (0.110, 1.037) | | | | |
| Age ≥60 vs <60 in Black | | | 3.493 (0.949, 12.859) | | | | |
| Age ≥60 vs <60 in White Non-Hisp | | | 0.287 (0.102, 0.809) | | | | |
| Age ≥60 vs <60 in White Hisp | | | 0.677 (0.330, 1.390) | | | | |
| Alcohol Ever vs Never in Male | | | 1.562 (0.685, 3.563) | | | | |
| Alcohol Ever vs Never in Female | | | 0.189 (0.053, 0.675) | | | | |
| Male vs Female in alcohol=Never | | | 0.213 (0.061, 0.750) | | | | |
| Male vs Female in alcohol=Ever | | | 1.758 (0.714, 4.332) | | | | |
| Logistic regression model included CD44-protein risk groups (5 categories, p<0.0001), age (≥60 vs.<60, p=0.060), gender (male vs. female, p=0.016), race/ethnicity (WNH and WH vs. Black, p=0.0027), alcohol (ever vs. never, p=0.0103), and interaction race/ethnicity×age (p=0.0138) and gender×alcohol (p=0.0063). Smoking (ever vs. never, p=0.844) and teeth removed (6 or more or all vs. 5 or less p=0.274) were not included to model since their inclusion did not improve model fit. | | | | | | | |

Table 11, Panel A, shows that a higher percentage of HPV+ subjects were in risk group 3, while a higher percentage of HPV- subjects were in risk group 4. Patients with stage IV disease are more common in risk groups 1 or 5 compared to patients with less advanced tumors (Table 13, Panel A).

**Table 13. SolCD44/Total Protein Test in Detecting and Determining Prognosis of HNSCC**

| **A. Percentage of Cases in Each Risk Group by HPV Status and Stage** | | | | | | |
|---|---|---|---|---|---|---|
| | HPV (%) | | Stage (%) | | | |
| Risk Group | Positive | Negative | I-II | III | IV | |
| | (n=31) | (n=48) | (n=26) | (n=24) | (n=100) | |
| 1 | 19.3 | 18.8 | 15.4 | 8.3 | 23 | |
| 2 | 3.2 | 0 | 3.8 | 0 | 3 | |
| 3 | 22.6 | 8.3 | 11.5 | 12.5 | 10 | |
| 4 | 22.6 | 41.7 | 46.2 | 66.7 | 26 | |
| 5 | 32.3 | 31.2 | 23.1 | 12.5 | 38 | |

| **B. Progression-Free and Overall Survival** | | | | | | |
|---|---|---|---|---|---|---|
| | **Univariate** | | | **Multivariate** | | |
| | **PFS (n= =137)** | | | **PFS (n= =136)¹** | | |
| Risk Group | n | HazardRatio (95%CI) | P-value | n | Hazard Ratio (95%CI) | P-value |
| 1 | 28 | 1.0 | | 28 | 1.0 | |
| 2 | 4 | 4.077 (1.051, 15.812) | 0.0421 | 4 | 3.863 (0.966, 15.445) | 0.0560 |
| 3 | 16 | 1.916 (0.694, 5.293) | 0.2098 | 15 | 1.808 (0.618, 5.294) | 0.2798 |
| 4 | 47 | 1.637 (0.692, 3.875) | 0.2621 | 47 | 1.896 (0.794, 4.528) | 0.1498 |
| 5 | 42 | 3.683 (1.616, 8.398) | 0.0019 | 42 | 3.588 (1.558, 8.265) | 0.0027 |

| **Risk Group Overall Survival(n=149)** | | | | **Overall Survival n=148) ²** | | |
|---|---|---|---|---|---|---|
| 1 | 29 | 1.0 | | 29 | 1.0 | |
| 2 | 4 | 3.291 (0.821, 13.189) | 0.0926 | 4 | 2.406 (0.579, 9.990) | 0.2268 |
| 3 | 16 | 0.916 (0.258, 3.259) | 0.8926 | 15 | 0.914 (0.251, 3.323) | 0.8912 |
| 4 | 53 | 1.406 (0.562, 3.516) | 0.4659 | 53 | 1.800 (0.710, 4.562) | 0.2157 |
| 5 | 47 | 2.869 (1.179, 6.980) | 0.0201 | 47 | 2.882 (1.165, 7.130) | 0.0220 |

| **C. Sensitivity and Specificity Using Regression Tree Model** | | | | | | |
|---|---|---|---|---|---|---|
| **SENSITIVITY** | | | | | | |
| 2012 Hospital-Based Cases (n=150) | | | 2006 Hospital-Based Cases (n=27) | | | |
| Stage I-II OC/OP | Stage I-III OC/OP | Stage I-IV OC/OP | Stage I-IV | OC/OP | | |
| 84.6% | 88% | 80.7% | 77.8% | | | |

| **SPECIFICITY** | | | | | | |
|---|---|---|---|---|---|---|
| 2012 Hospital-Based Controls (n=150) | 2006 Hospital-Based Controls (n=39) | Community-Based Controls (n=150) Single Sample | Community-Based Controls (n=95) Two samples | Normal Volunteers (n=21) | | |
| 48.7% | 56.4% | 74% | 95% | 95.2% | | |

| | | | | | | |
|---|---|---|---|---|---|---|
| ¹Cox's proportional hazards model for PFS included risk group (5 categories, p=0.0181), stage (III/IV vs. I/II, p=0.0117), race (Black vs other, p=0.0066), ethnicity (Non-Hispanic vs. Hispanic, p=0.5964), gender (Female vs. Male, p=0.5779), and age in years (p=0.2112). ² Cox's proportional hazards model for OS included risk group (5 categories, p=0.0750), stage (III/IV vs. I/II, p=0.0315), race (Black vs other, p=0.0041), ethnicity (Non-Hispanic vs. Hispanic, p=0.1091), gender (Female vs. Male, p=0.2663), and age in years (p=0.0725). | | | | | | |

### Prognostic Significance of Markers

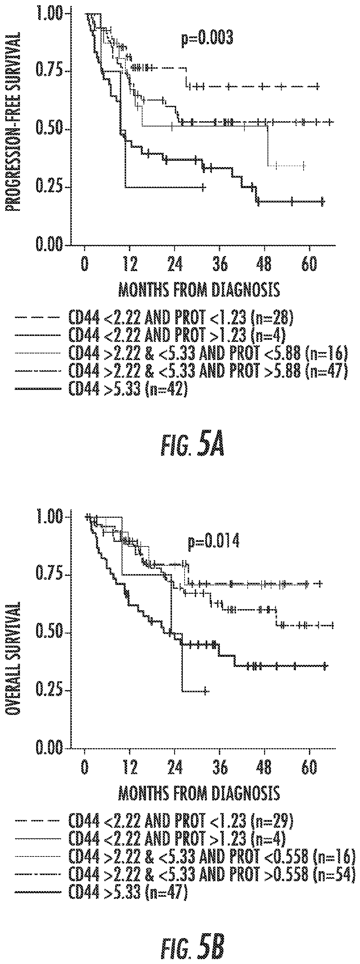

Kaplan-Meier curves for progression-free survival (PFS) and overall survival (OS) by risk group are shown in Figure 5A and 5B. Unadjusted and adjusted estimates of hazard ratios (HRs) for PFS and OS by risk groups are shown in Table 13, Panel B. Based on multivariate analysis with adjustment for tumor stage, age, gender race and ethnicity, hospital-based cases that fell into risk group 5 (high CD44) had reduced PFS (adjusted HR=3.588, 95%CI: 1.558, 8.265, p=0.0027) and OS (adjusted HR=2.882, 95%CI: 1.165, 7.130, p=0.0220) compared with cases in risk group 1. Risk group 2 had borderline association with decreased PFS (HR=3.863, 95%CI: 0.966, 15.445, p=0.056) and no significant difference in OS (HR=2.406, 95%CI: 0.579, 9.990, p=0.2268) compared to cases in risk group 1; however, this group included only 4 cases.

### Sensitivity and Specificity

Sensitivity for predicting case status was 80.7% (Table 13C). This was validated in the 2006 hospital-based study(sensitivity 77.8%).33 For the 2012 hospital-based cases, sensitivity reaches 88% for stage I-III cancers (Table 13C). Specificity was validated in hospital-based controls, a community at high-risk for HNSCC (n=150, 98% smokers, poor access to healthcare) and normal volunteers who were predominantly non-smokers (Table 13C). The specificity was greatest in the normal volunteer cohort (95.2%). On further evaluation it was found that over 10% of the hospital-based controls versus less than 1% of the community cohort had a history of prior cancer outside the UADT. Hospital-based controls with prior history of cancer had significantly higher solCD44 levels compared to controls without prior cancer (p<0.05).

### Reversal of Risk

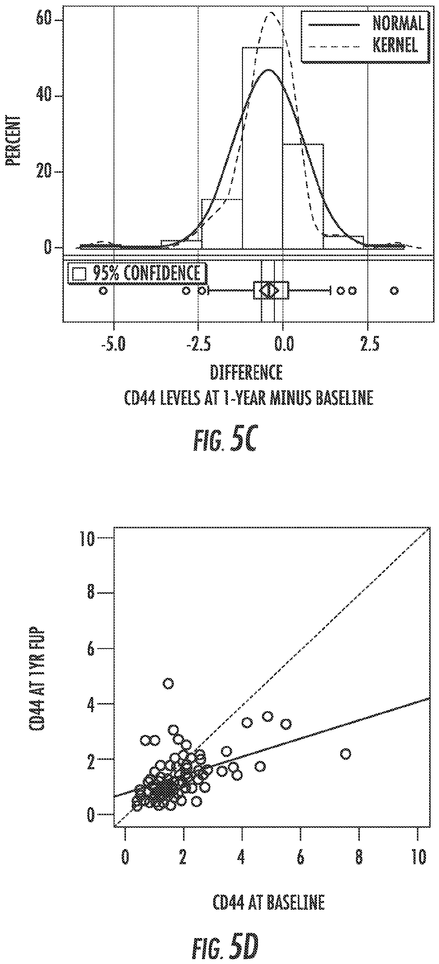

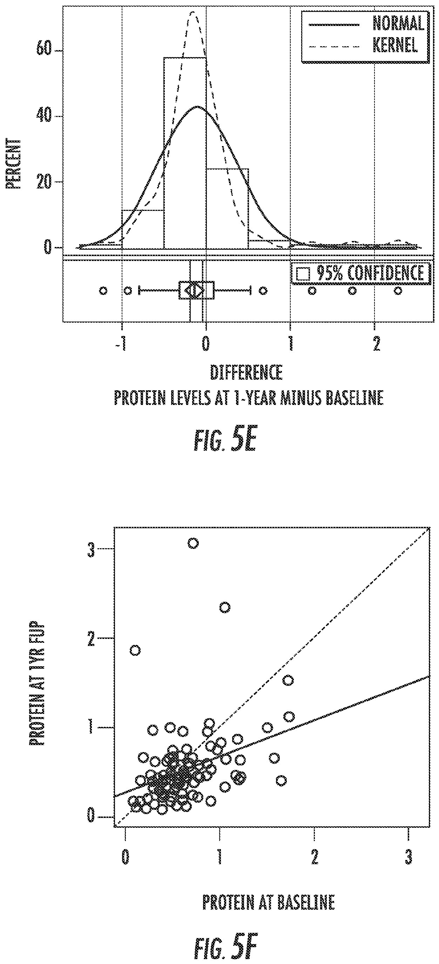

A total of 95 patients in the community-based cohort provided baseline and annual follow-up collections. The distribution of changes in CD44 and protein levels over 1 year are shown in Figure 5C and 5E, respectively. Average CD44 levels were 1.829 ng/mL at baseline and 1.390 ng/mL at 1 year follow-up. The average annual drop in CD44 levels of - 0.439 ng/mL (24%) was significant (p<.0001). Linear regression analysis confirmed a significant linear trend for lower CD44 values (R2=0.227, intercept=0.785 (p<.0001), slope=0.331 (p<.0001)), (Figure 5D). Mean protein levels also dropped significantly from 0.644 to 0.543 mg/mL (p=0.036) with confirmation by linear regression analysis (R2=0.108, intercept=0.284 (p=0.002), slope=0.402 (p<.0001)), (Figure 5F). To determine if these changes were due to variation in assay conditions over the course of the year, a baseline second aliquot (baseline 2) was run on the same plate as the annual follow-up collection with 81 such pairs for each assay (protein and CD44). The drop in levels between baseline 2 and annual follow-up was significant only for CD44 (CD44: -0.296ng/mL, p=0.023; protein: -0.013 mg/mL, p=0.796) while linear regression showed a significant trend towards lower numbers for both markers (CD44: R2=0.227, intercept=0.882 (p<.0001), slope=0.288 (p<.0001); protein. R2=0.155, intercept=0.256 (p=0.008), slope=0.534 (p<.0001). The two baselines were compared by linear regression of baseline 2 on baseline 1. For CD44, correlation was high (R=0.899), intercept was not significantly different from zero (p=0.150) and slope was not significantly different from 1 (p=0.881), indicating that the two were equivalent. For protein, correlation was also high (R=0.925), intercept was not significant difference from zero (p=0.712), but slope was significantly different from 1 (0.85, p<.001), indicating that the differences between baselines for protein were not within the expected random variation.

Of 22 community subjects in the moderate (risk group 4, n=14) or high-risk risk group 2, n=1, risk group 3 n=5 and risk group 5 n=2) only 5 remained in an at-risk category (risk group 4, n=3, risk group 2 n=1, risk group 5 n=1) after 1 year follow-up.

### Role of CD44 in Detecting Oral Cancer or Cancers at Other Sites

2 subjects fell into an elevated risk category (both risk group 4) and developed early HNSCC (lip and carcinoma in situ of the larynx) in follow-up. One control, excluded because of bladder cancer, had solCD44 levels of 14 ng/mL and protein levels of 1.5 mg/mL. Another control, excluded due to concern for oral pre-malignancy, had solCD44 levels of 4.8 ng/mL and protein levels of 1.3 mg/mL and went on to develop lung cancer. A patient from the community-based study developed lung cancer and had elevated levels (CD44 =3.975 ng/mL, protein=0.656 mg/mL) 14 months before diagnosis.

### Discussion

The methods disclosed herein show that an inexpensive, noninvasive screening tool based on CD44 and protein is able to accurately distinguish oral cancer cases from controls. Frequency matching, unique herein, prevents confounding by covariates such as tobacco use or socioeconomic status. Over 85 million individuals in the United States are at risk for oral cancer, but very few of these vulnerable individuals actually receive an oral exam. The oral rinse molecular test described here could revolutionize oral cancer screening, by providing a simple and reliable measure of oral cancer risk that alerts primary care providers and dentists to the individuals most in need of skilled oral exam. The CD44 ELISA assay and protein test have already been converted to a lateral flow test strip prototype. Thus mass screenings are feasible.

The study also shows that high CD44 is associated with poor PFS and OS. These markers can be useful in guiding therapy (Ang et al. N Engl J Med. 2010 1;363:24-35) The test can detect earlier stage (I-III) oral cancer better than late stage (IV) disease. The role of CD44 can be important for tumor initiation.

Two patients in this study who were thought to be false positives developed HNSCC during the follow-up period (Franzmann et al. Cancer Epidemiol Biomarkers Prev 2007;16:1348-55). Further, subjects with other smoking associated tumors including lung and bladder also had elevated CD44 levels. Thus "false positives" can actually be true positives for occult oral disease or other cancers with related risk factors. Since CD44 is a tumor initiation factor, levels can go down if risk factors decrease and occult lesions disappear. The data shows that individuals who stayed in the community screening program for a year underwent a significant decrease in CD44 levels. All subjects received education on smoking cessation and access to resources to assist them in improving oral hygiene and nutrition. Thus behavioral changes may have resulted in decreased levels.

## Claims

1. A method of determining a risk of head and neck squamous cell cancer in a subject using a kit comprising: saline solution; a cup for receiving an oral saline rinse; at least one antibody that specifically binds CD44; a reagent for determining total protein concentration; and reference levels for soluble CD44 (solCD44) and total protein, the method comprising:
a) measuring a test amount of solCD44 in a sample of an oral saline rinse obtained from the subject in the cup, using the at least one antibody that specifically binds CD44;
b) measuring a test amount of total protein in the sample using the reagent for determining total protein concentration; and
c) determining the risk of head and neck squamous cell cancer in the subject by determining whether the test amount of solCD44 and the test amount of total protein are above or below the reference levels of solCD44 and total protein, wherein the reference levels of solCD44 are about 2.2 ng/mL and about 5.3 ng/mL and the reference levels of total protein are about 1.2 mg/mL and about 0.6 mg/mL, wherein the reference levels of solCD44 and total protein delimit different, statistically significant risks for the cancer and wherein the term about means within 5% of the specified value.

2. The method of claim 1, wherein the step of determining the risk of head and neck squamous cell cancer in the subject comprises determining whether:
(i) the test amount of solCD44 is below about 2.2 ng/mL and the test amount of total protein is below about 1.2 mg/mL;
(ii) the test amount of solCD44 is above or equal to about 2.2 ng/mL but below about 5.3 ng/mL and the test amount of total protein is above or equal to about 0.6 mg/mL;
(iii) the test amount of solCD44 is below about 2.2 ng/mL and the test amount of total protein is above or equal to about 1.2 mg/mL;
(iv) the test amount of solCD44 is above or equal to about 2.2 ng/mL but below about 5.3 ng/mL and the test amount of total protein is below about 0.6 mg/mL; or
(v) the test amount of solCD44 is above about 5.3 ng/mL, wherein the term about means within 5% of the specified value.

3. The method of claim 1 or 2, wherein the step of determining the risk of head and neck squamous cell cancer in the subject comprises determining whether:
(i) the test amount of solCD44 is below 2.22 ng/mL and the test amount of total protein is below 1.23 mg/mL;
(ii) the test amount of solCD44 is above or equal to 2.22 ng/mL but below 5.33 ng/mL and the test amount of total protein is above or equal to 0.558 mg/mL;
(iii) the test amount of solCD44 is below 2.22 ng/mL and the test amount of total protein is above or equal to 1.23 mg/mL;
(iv) the test amount of solCD44 is above or equal to 2.22 ng/mL but below 5.33 ng/mL and the test amount of total protein is below 0.558 mg/mL; or
(v) the test amount of solCD44 is above 5.33 ng/mL.

4. The method of claim 1, wherein the kit further comprises at least one antibody that binds p16 INK4a and the method comprises a step of measuring p16 INK4a in the sample using the at least one antibody that binds p16 INK4a.

5. The method of claim 4, wherein the at least one antibody that specifically binds p16 INK4a comprises the idiotype of the E6H4 antibody clone.

6. The method of any one of claims 1 to 5, wherein the kit further comprises a CD44 reference sample, a p16 reference sample, or a combination thereof.

7. The method of any one of claims 1 to 6, wherein the kit further comprises one or more colorimetric agents for the detection of the antibody that specifically binds p16 INK4a, the antibody that specifically binds CD44, or a combination thereof.

8. The method of any one of claims 1 to 7, wherein the kit is a lateral flow immunoassay.

9. The method of any one of claims 1 to 8, wherein the kit comprises a multi-well plate optionally coated with the antibody that specifically binds p16 INK4a, the antibody that specifically binds CD44, or a combination thereof.

10. The method of any one of claims 1 to 9 wherein the test amount of solCD44 is normalized to the test amount of total protein before determining the risk of head and neck squamous cell cancer.

11. The method of any one of the previous claims, wherein the oral saline rinse is obtained at least one hour after the subject has eaten, smoked, and drank.

12. The method of any one of the previous claims, wherein:
(i) the subject is selected to provide the oral saline rinse based on the subject's age, race, history of alcohol consumption, history of tobacco use, history of cancer, and/or positive result of a human papilloma virus (HPV) assay; and/or
(ii) the subject has no clinically visible malignancy or premalignancy; and/or
(iii) wherein the subject is selected for treatment through surgery, radiation, or chemotherapy, or a combination thereof, based on the identified risk.

13. The method of any one of the previous claims, wherein:
(a) an immunoassay is used to measure the test amount of solCD44 in the sample, preferably wherein the immunoassay used to measure the test amount of solCD44 in the sample is an Enzyme Linked Immunosorbent Assay (ELISA) or Lateral Flow Assay; and/or
(b) a Lowry or modified-Lowry protein assay is used to measure the test amount of total protein in the sample.

14. The method of any one of the previous claims, further comprising:
(i) providing risk factor management counseling to the subject, preferably wherein the risk factor management counseling comprises a smoking cessation program; and/or
(ii) repeating steps a) through c) at regular time intervals, preferably wherein steps a) through c) are repeated every three to six months; and/or
(iii) step d) referring the subject to a cancer specialist; and/or
(iv) measuring the level of at least one of: hyaluronic acid (HA) and hyaluronidase (HAase) in the sample.

## Patentansprüche

1. Verfahren zur Bestimmung eines Risikos von Plattenepithelkarzinom im Kopf- und Halsbereich bei einer Versuchsperson unter Verwendung eines Kits, umfassend: Kochsalzlösung; einen Becher zur Aufnahme einer oralen Kochsalzlösungsspülung; mindestens einen Antikörper, der spezifisch an CD44 bindet; ein Reagens zur Bestimmung der Gesamtproteinkonzentration; und Referenzwerte für lösliches CD44 (solCD44) und Gesamtprotein, wobei das Verfahren umfasst:
a) Messung einer Testmenge an solCD44 in einer Probe einer oralen Kochsalzlösungsspülung, die von der Versuchsperson in dem Becher gewonnen wurde, unter Verwendung des mindestens einen Antikörpers, der CD44 spezifisch bindet;
b) Messung einer Testmenge an Gesamtprotein in der Probe unter Verwendung des Reagens zur Bestimmung der Gesamtproteinkonzentration; und
c) Bestimmung des Risikos von Plattenepithelkarzinom im Kopf- und Halsbereich bei der Versuchsperson, indem bestimmt wird, ob die Testmenge an solCD44 und die Testmenge an Gesamtprotein über oder unter den Referenzwerten von solCD44 und Gesamtprotein liegen, wobei die Referenzwerte von solCD44 etwa 2,2 ng/mL und etwa 5.3 ng/mL und die Referenzwerte von Gesamtprotein etwa 1,2 mg/mL und etwa 0,6 mg/mL betragen, wobei die Referenzwerte von solCD44 und Gesamtprotein unterschiedliche, statistisch signifikante Risiken für den Krebs umgrenzen und wobei der Begriff 'etwa' innerhalb von 5% des spezifizierten Wertes bedeutet.

2. Verfahren nach Anspruch 1, wobei der Schritt der Bestimmung des Risikos von Plattenepithelkrebs im Kopf- und Halsbereich bei der Versuchsperson die Bestimmung umfasst, ob: die Testmenge an solCD44 unter etwa 2,2 ng/mL und die Testmenge an Gesamtprotein unter etwa 1,2 mg/mL ist;
(i) die Testmenge an solCD44 unter etwa 2,2 ng/mL und die Testmenge an Gesamtprotein unter etwa 1,2 mg/mL ist;
(ii) die Testmenge an solCD44 über oder gleich etwa 2,2 ng/mL, aber unter etwa 5,3 ng/mL ist und die Testmenge an Gesamtprotein über oder gleich etwa 0,6 mg/mL ist;
(iii) die Testmenge an solCD44 unter etwa 2,2 ng/mL ist und die Testmenge an Gesamtprotein über oder gleich etwa 1,2 mg/mL ist;
(iv) die Testmenge an solCD44 über oder gleich etwa 2,2 ng/mL, aber unter etwa 5,3 ng/mL ist und die Testmenge an Gesamtprotein über oder gleich etwa 0,6 mg/mL ist; oder
(v) die Testmenge an solCD44 über etwa 5,3 ng/mL ist, wobei der Begriff 'etwa' innerhalb von 5% des spezifizierten Wertes bedeutet.

3. Verfahren nach Anspruch 1 oder 2, wobei der Schritt der Bestimmung des Risikos von Plattenepithelkrebs im Kopf- und Halsbereich bei der Versuchsperson die Bestimmung umfasst, ob:
(i) die Testmenge an solCD44 unter etwa 2,22 ng/mL und die Testmenge an Gesamtprotein unter etwa 1,23 mg/mL ist;
(ii) die Testmenge an solCD44 über oder gleich etwa 2,22 ng/mL, aber unter etwa 5,33 ng/mL ist und die Testmenge an Gesamtprotein über oder gleich etwa 0,558 mg/mL ist;
(iii) die Testmenge an solCD44 unter etwa 2,22 ng/mL ist und die Testmenge an Gesamtprotein über oder gleich etwa 1,23 mg/mL ist;
(iv) die Testmenge an solCD44 über oder gleich etwa 2,22 ng/mL, aber unter etwa 5,33 ng/mL ist und die Testmenge an Gesamtprotein über oder gleich etwa 0,558 mg/mL ist; oder
(v) die Testmenge an solCD44 über 5,33 ng/mL ist.

4. Verfahren nach Anspruch 1, wobei das Kit weiter mindestens einen Antikörper umfasst, der an p16 INK4a bindet, und das Verfahren einen Schritt zur Messung von p16 INK4a in der Probe unter Verwendung des mindestens einen Antikörpers, der an p16 INK4a bindet, umfasst.

5. Verfahren nach Anspruch 4, wobei der mindestens eine Antikörper, der spezifisch an p16 INK4a bindet, den Idiotyp des E6H4-Antikörperklons umfasst.

6. Verfahren nach einem der Ansprüche 1 bis 5, wobei das Kit weiter eine CD44-Referenzprobe, eine p16-Referenzprobe oder eine Kombination davon umfasst.

7. Verfahren nach einem der Ansprüche 1 bis 6, wobei das Kit weiter ein oder mehrere kolorimetrische Mittel zum Nachweis des Antikörpers, der spezifisch an p16 INK4a bindet, des Antikörpers, der spezifisch an CD44 bindet, oder einer Kombination davon umfasst.

8. Methode nach einem der Ansprüche 1 bis 7, wobei das Kit ein Lateral-Flow-Immunoassay ist.

9. Verfahren nach einem der Ansprüche 1 bis 8, wobei das Kit eine Multiwellplatte umfasst, die optional mit dem Antikörper, der spezifisch an p16 INK4a bindet, dem Antikörper, der spezifisch an CD44 bindet, oder einer Kombination davon beschichtet ist.

10. Verfahren nach einem der Ansprüche 1 bis 9, wobei die Testmenge an solCD44 auf die Testmenge an Gesamtprotein normalisiert wird, bevor das Risiko eines Plattenepithelkarzinoms im Kopf- und Halsbereich bestimmt wird.

11. Verfahren nach einem der vorstehenden Ansprüche, wobei die orale Kochsalzlösungsspülung mindestens eine Stunde, nachdem die Versuchsperson gegessen, geraucht und getrunken hat, gewonnen wird.

12. Verfahren nach einem der vorstehenden Ansprüche, wobei:
(i) die Versuchsperson ausgewählt wird, um die orale Kochsalzlösungsspülung basierend auf dem Alter, der Rasse, der Vorgeschichte an Alkoholkonsum, der Vorgeschichte an Tabakkonsum, der Vorgeschichte an Krebs und/oder eines positiven Ergebnisses eines Human-Papillomavirus- (HPV-) Assays der Versuchsperson bereitzustellen; und/oder
(ii) wobei die Versuchsperson keine klinisch sichtbare maligne oder prämaligne Erkrankung aufweist; und/oder
(iii) wobei das Subjekt durch Chirurgie, Bestrahlung oder Chemotherapie oder eine Kombination davon, basierend auf dem identifizierten Risiko, für die Behandlung ausgewählt wird.

13. Verfahren nach einem der vorstehenden Ansprüche, wobei:
(a) ein Immunoassay verwendet wird, um die Testmenge an solCD44 in der Probe zu messen, bevorzugt wobei der Immunoassay, der verwendet wird, um die Testmenge an solCD44 in der Probe zu messen, ein Enzyme Linked Immunosorbent Assay (ELISA) oder Lateral-Flow-Assay ist; und/oder
(b) ein Lowry- oder Modified-Lowry-Protein-Assay verwendet wird, um die Testmenge an Gesamtprotein in der Probe zu messen.

14. Verfahren nach einem der vorstehenden Ansprüche, weiter umfassend:
(i) Bereitstellung von Beratung zum Risikofaktorenmanagement für die Versuchsperson, wobei die Beratung zum Risikofaktorenmanagement bevorzugt ein Raucherentwöhnungsprogramm umfasst; und/oder
(ii) Wiederholung der Schritte a) bis c) in regelmäßigen Zeitabständen, wobei die Schritte a) bis c) bevorzugt alle drei bis sechs Monate wiederholt werden; und/oder
(iii) Schritt d) Überweisung der Versuchsperson an einen Krebsspezialisten; und/oder
(iv) Messung des Niveaus von mindestens einem von: Hyaluronsäure (HA) und Hyaluronidase (HAase) in der Probe.

## Revendications

1. Procédé de détermination d'un risque de cancer à cellules squameuses de la tête et du cou chez un patient au moyen d'un kit comprenant: une solution saline; une coupelle pour recevoir un rinçage salin oral ; au moins un anticorps qui se lie spécifiquement à la CD44 ; un réactif pour déterminer une concentration en protéine totale ; et des niveaux de référence pour la CD44 soluble (solCD44) et la protéine totale, le procédé comprenant les étapes consistant à :
a) mesurer une quantité de test de solCD44 dans un échantillon d'un rinçage salin oral obtenu à partir du sujet dans la coupelle, en utilisant le au moins un anticorps qui se lie spécifiquement à la CD44 ;
b) mesurer une quantité de test de protéine totale dans l'échantillon en utilisant le réactif pour déterminer la concentration en protéine totale ; et
c) déterminer le risque de cancer à cellules squameuses de la tête et du cou chez le patient en déterminant si la quantité de test de solCD44 et la quantité de test de protéine totale sont supérieures ou inférieures aux niveaux de référence de solCD44 et de protéine totale, dans lequel les niveaux de référence de solCD44 sont d'environ 2,2 ng/ml et d'environ 5,3 ng/ml et les niveaux de référence de protéine totale sont d'environ 1,2 mg/ml et d'environ 0,6 mg/ml, dans lequel les niveaux de référence de solCD44 et de protéine totale délimitent des risques différents et statistiquement significatifs pour le cancer et dans lequel le terme environ signifie dans la limite de 5 % de la valeur spécifiée.

2. Procédé selon la revendication 1, dans lequel l'étape de détermination du risque de cancer à cellules squameuses de la tête et du cou chez le patient consiste à déterminer si :
(i) la quantité de test de solCD44 est inférieure à environ 2,2 ng/ml et la quantité de test de protéine totale est inférieure à environ 1,2 mg/ml ;
(ii) la quantité de test de solCD44 est supérieure ou égale à environ 2,2 ng/ml mais inférieure à environ 5,3 ng/ml et la quantité de test de protéine totale est supérieure ou égale à environ 0,6 mg/ml ;
(iii) la quantité de test de solCD44 est inférieure à environ 2,2 ng/ml et la quantité de test de protéine totale est supérieure ou égale à environ 1,2 mg/ml ;
(iv) la quantité de test de solCD44 est supérieure ou égale à environ 2,2 ng/ml mais inférieure à environ 5,3 ng/ml et la quantité de test de protéine totale est inférieure à environ 0,6 mg/ml ; ou
(v) la quantité de test de solCD44 est supérieure à environ 5,3 ng/ml, dans lequel le terme environ signifie dans la limite de 5 % de la valeur spécifiée.

3. Procédé selon la revendication 1 ou 2, dans lequel l'étape de détermination du risque de cancer à cellules squameuses de la tête et du cou chez le patient consiste à déterminer si :
(i) la quantité de test de solCD44 est inférieure à 2,22 ng/ml et la quantité de test de protéine totale est inférieure à 1,23 mg/ml ;
(ii) la quantité de test de solCD44 est supérieure ou égale à 2,22 ng/ml mais inférieure à 5,33 ng/ml et la quantité de test de protéine totale est supérieure ou égale à 0,558 mg/ml ;
(iii) la quantité de test de solCD44 est inférieure à 2,22 ng/ml et la quantité de test de protéine totale est supérieure ou égale à 1,23 mg/ml ;
(iv) la quantité de test de solCD44 est supérieure ou égale à 2,22 ng/ml mais inférieure à 5,33 ng/ml et la quantité de test de protéine totale est inférieure à 0,558 mg/ml ; ou
(v) la quantité de test de solCD44 est supérieure à 5,33 ng/ml.

4. Procédé selon la revendication 1, dans lequel le kit comprend en outre au moins un anticorps qui se lie à p16 INK4a et le procédé comprend une étape de mesure de p16 INK4a dans l'échantillon en utilisant le au moins un anticorps qui se lie à p16 INK4a.

5. Procédé selon la revendication 4, dans lequel le au moins un anticorps qui se lie spécifiquement à p16 INK4a comprend l'idiotype du clone d'anticorps E6H4.

6. Procédé selon l'une quelconque des revendications 1 à 5, dans lequel le kit comprend en outre un échantillon de référence de CD44, un échantillon de référence de p16 ou une combinaison de ceux-ci.

7. Procédé selon l'une quelconque des revendications 1 à 6, dans lequel le kit comprend en outre un ou plusieurs agents colorimétriques pour la détection de l'anticorps qui se lie spécifiquement à p16 INK4a, l'anticorps qui se lie spécifiquement à CD44, ou une combinaison de ceux-ci.

8. Procédé selon l'une quelconque des revendications 1 à 7, dans lequel le kit est un immunodosage à écoulement latéral.

9. Procédé selon l'une quelconque des revendications 1 à 8, dans lequel le kit comprend une plaque à puits multiples facultativement revêtue de l'anticorps qui se lie spécifiquement à p16 INK4a, de l'anticorps qui se lie spécifiquement à CD44, ou d'une combinaison de ceux-ci.

10. Procédé selon l'une quelconque des revendications 1 à 9, dans lequel la quantité de test de solCD44 est normalisée par rapport à la quantité de test de protéine totale avant de déterminer le risque de cancer à cellules squameuses de la tête et du cou.

11. Procédé selon l'une quelconque des revendications précédentes, dans lequel le rinçage salin oral est obtenu au moins une heure après que le patient a mangé, fumé et bu.

12. Procédé selon l'une quelconque des revendications précédentes, dans lequel :
(i) le patient est sélectionné pour fournir le rinçage salin oral sur la base de l'âge, de la race, des antécédents de consommation d'alcool, des antécédents de tabagisme, des antécédents de cancer et/ou d'un résultat positif d'un test de dépistage du virus du papillome humain (VPH) du patient ; et/ou
(ii) le patient n'a aucune tumeur maligne ou prémaligne cliniquement visible ; et/ou
(iii) dans lequel le patient est sélectionné pour un traitement par chirurgie, radiothérapie ou chimiothérapie, ou une combinaison de ceux-ci, sur la base du risque identifié.

13. Procédé selon l'une quelconque des revendications précédentes, dans lequel :
(a) un immunodosage est utilisé pour mesurer la quantité de test de solCD44 dans l'échantillon, de préférence dans lequel l'immunodosage utilisé pour mesurer la quantité de test de solCD44 dans l'échantillon est un dosage d'immunosorbant lié à une enzyme (ELISA) ou un dosage à écoulement latéral ; et/ou
(b) un dosage de protéine de Lowry ou de Lowry modifié est utilisé pour mesurer la quantité de test de protéine totale dans l'échantillon.

14. Procédé selon l'une quelconque des revendications précédentes, comprenant en outre les étapes consistant à :
(i) fournir au sujet un conseil en gestion des facteurs de risque, de préférence dans lequel le conseil en gestion des facteurs de risque comprend un programme de sevrage tabagique ; et/ou
(ii) répéter les étapes a) à c) à des intervalles de temps réguliers, de préférence dans lequel les étapes a) à c) sont répétées tous les trois à six mois ; et/ou
(iii) une étape d) consistant à renvoyer le patient vers un cancérologue ; et/ou
(iv) mesurer le niveau d'au moins l'un parmi : l'acide hyaluronique (HA) et la hyaluronidase (HAase) dans l'échantillon.
